# EUROPEAN PATENT APPLICATION

(11) **EP 4 520 838 A1**
(43) Date of publication of application: **12.03.2025**
(21) Application number: 23911608.0
(22) Date of filing: 07.12.2023
(51) Int. Cl.: C12P 19/00, A23L 33/18, A61K 35/12, A61K 35/32, A61K 35/37, A61K 35/42, A61K 35/44, A61K 35/57, A61K 35/60, A61P 43/00, C07K 14/78

(54) **PROTEOGLYCAN PRODUCTION METHOD AND PROTEOGLYCAN**

(30) Priority: 28.12.2022 JP 2022211853
(71) Applicant: National University Corporation Tottori University, Tottori-shi, Tottori 680-8550 (JP); Ichimaru Pharcos Co., Ltd., Motosu-shi, Gifu 501-0475 (JP)
(72) Inventor: TAMURA Junichi, Tottori-shi, Tottori 680-8550 (JP); OKUDA Naoko, Tottori-shi, Tottori 680-8550 (JP); MASUTANI Teruaki, Motosu-shi, Gifu 501-0475 (JP); TAKAHASHI Tatsuji, Motosu-shi, Gifu 501-0475 (JP)
(74) Representative: Hasegawa, Kan
(86) International application number: PCT/JP2023/043810
(87) International publication number: WO 2024/142827

(57) **Abstract**

The present disclosure produces proteoglycan in a simpler manner than known methods for producing proteoglycan, and provides a method capable of producing proteoglycan with a core protein and sugar chains bonded. The method for producing proteoglycan of the present disclosure includes: extracting an extract comprising proteoglycan from a tissue of an animal. The proteoglycan comprises a core protein and a sugar chain, and the core protein and the sugar chain are bonded.

## Description

### TECHNICAL FIELD

The present disclosure relates to a proteoglycan production method and proteoglycan.

### BACKGROUND ART

Proteoglycans are molecules that constitute an extracellular matrix with other components such as collagen and hyaluronic acid. The proteoglycans are known to have excellent water retention and many physiological functions such as an anti-inflammatory action, an action of accelerating hyaluronic acid synthesis, and a cell proliferation promoting action. Thus, various studies have been made to use the proteoglycans for cosmetics, foods, and beverages.

However, known methods for producing proteoglycan are disadvantageous because a substance harmful to animals including humans, such as EDTA, is used, the production process is complicated to remove the harmful substance, and the yield of proteoglycan is reduced (Non-Patent Documents 1 and 2).

### CITATION LIST

### PATENT DOCUMENTS

Non-Patent Document 1: Kakizaki I, Tatara Y, Majima M, Kato Y, Endo M. Identification of proteoglycan from salmon nasal cartilage. Arch Biochem Biophys. 2011 Feb 1;506(1):58-65. doi: 10.1016/j.abb.2010.10.025. Epub 2010 Nov 5. PMID: 21056541.

Non-Patent Document 2: Kakizaki I, Mineta T, Sasaki M, Tatara Y, Makino E, Kato Y. Biochemical and atomic force microscopic characterization of salmon nasal cartilage proteoglycan. Carbohydr Polym. 2014 Mar 15; 103:538-49. doi: 10.1016/j.carbpol.2013.12.083. Epub 2014 Jan 7. Erratum in: Carbohydr Polym. 2015 Jan 22;115:805. PMID: 24528764.

### SUMMARY OF THE INVENTION

### TECHNICAL PROBLEM

In view of the foregoing, for example, an object of the present disclosure is to produce proteoglycan in a simpler manner than by known methods for producing proteoglycan, and to produce proteoglycan with a core protein and sugar chains bonded.

### SOLUTION TO THE PROBLEM

In order to achieve the object, the present disclosure provides a method for producing proteoglycan (will be hereinafter also referred to as a "production method"). The method includes: extracting an extract comprising proteoglycan from a tissue of an animal, the proteoglycan comprises a core protein and a sugar chain, and the core protein and the sugar chain are bonded.

A composition of the present disclosure comprises proteoglycan. The proteoglycan has a peak top molecular weight of 400,000 to 1,200,000.

### ADVANTAGES OF THE INVENTION

The present disclosure can produce proteoglycan in a simpler manner than known methods for producing proteoglycan, and can produce proteoglycan with a core protein and sugar chains bonded.

### BRIEF DESCRIPTION OF THE DRAWINGS

FIG. 1 illustrates major disaccharide structures of chondroitin sulfates.
FIG. 2, (A) to (E) are graphs of the results of analyses of proteoglycan solids by high performance liquid chromatography (HPLC) conducted in Example 1.
FIG. 3 is a graph of the results of cell proliferation tests of chondroitin sulfate A conducted in Example 1.
FIG. 4, (A) to (F) are graphs of the results of cell proliferation tests conducted in Example 1 on proteoglycans obtained in Example 1(1).
FIG. 5, (A) to (D) are graphs of the results of HPLC of disaccharide structures of proteoglycans conducted in Example 3.
FIG. 6, (A) to (D) are graphs of the results of HPLC of disaccharide structures of proteoglycans conducted in Example 3.
FIG. 7 is a photograph of decorin detected in Example 3.

### DESCRIPTION OF EMBODIMENTS

The present disclosure will be described in detail below by way of examples. Hereinafter, unless otherwise specified, each of the examples can be incorporated by reference into the description of other examples.

### <Definitions>

As used herein, "proteoglycan" refers to a molecule (glycoprotein) having a protein (a core protein) to which glycosaminoglycans (GAGs, also referred to as "polysaccharides" or "sugar chains") are covalently bonded. The proteoglycan exists in, for example, an extracellular matrix of skin, organs, and cartilage. The glycosaminoglycans are generally known as sugar chains having a long-chain structure with no branch structures. Examples of the proteoglycan include: aggrecan family (also referred to as lectican family or hyalectan family) such as aggrecan, versican, neurocan, and brevican; small leucine-rich proteoglycans (SLRPs) family such as biglycan, decorin, fibromodulin, lumican, PG-Lb (epiphycan), keratocan, and mimecan; proteoglycans of a basement membrane such as perlecan, agrin, and bamacan; and other proteoglycans such as testican, biglycan, serglycin, syndecan, dystroglycan, claustrin, glypican, and keratocan. The proteoglycans can be classified into chondroitin sulfate proteoglycans, dermatan sulfate proteoglycans, heparan sulfate proteoglycans, and keratan sulfate proteoglycans depending on, for example, the type of GAGs bonded to the protein.

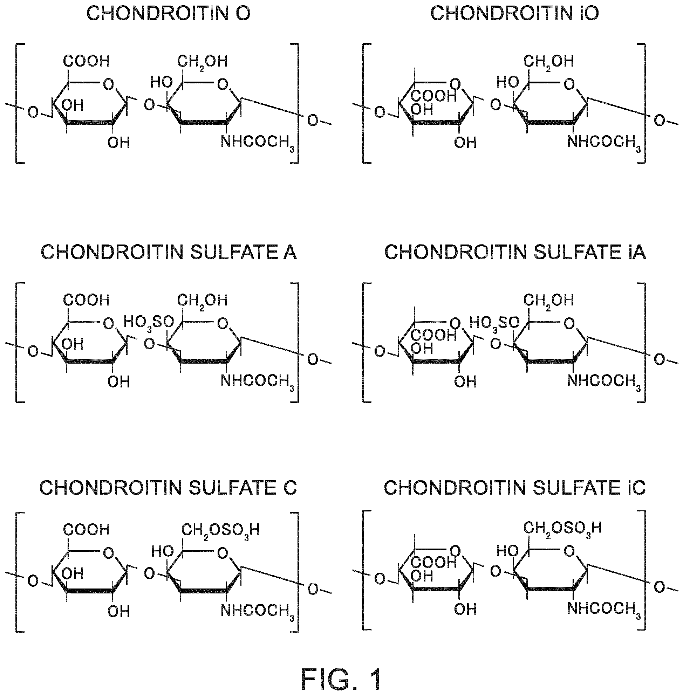

Examples of the GAGs include chondroitin, chondroitin sulfate (CS), dermatan sulfate (DS, chondroitin sulfate B), heparan sulfate, heparin, and keratan sulfate. Examples of the chondroitin include an O-sugar chain having a main disaccharide structure of glucuronic acid and acetylgalactosamine, and an iO-sugar chain having a main disaccharide structure of iduronic acid and acetylgalactosamine (they may also be referred to as "chondroitin sulfate O" and "chondroitin sulfate iO"). Chondroitin sulfate (CS) is composed of a sugar chain of two repeating sugars, glucuronic acid and acetylgalactosamine, and a sulfate group added to the sugar chain. Examples of the chondroitin sulfate include chondroitin sulfate A (type A) having a main disaccharide structure of glucuronic acid and acetylgalactosamine 4-sulfate, and chondroitin sulfate C (type C) having a main disaccharide structure of glucuronic acid and acetylgalactosamine 6-sulfate. Dermatan sulfate (DS) is composed of a sugar chain of two repeating sugars, iduronic acid and acetylgalactosamine, and a sulfate group added to the sugar chain. Examples of dermatan sulfate include chondroitin sulfate iA (type iA) having a main disaccharide structure of iduronic acid and acetylgalactosamine 4-sulfate, and chondroitin sulfate iC (type iC) having a main disaccharide structure of iduronic acid and acetylgalactosamine 6-sulfate. Each of the chondroitin sulfates has, for example, a main disaccharide structure shown in FIG. 1. In FIG. 1, the sulfate group (sulfo group) is bonded to a hydrogen atom, but the present disclosure is not limited to this example. The sulfate group of the GAG may be ionized by elimination of a hydrogen atom, or may form a salt, for example.

As used herein, "extraction/extracted" means taking a particular component out of an object and/or a state in which a particular component has been taken out of an object. The extraction can be performed using an extraction liquid such as a solvent. The "extraction" can be carried out by, for example, at least one extraction step. In the present specification, a substance comprising a component taken out of the subject is referred to as an extract. A liquid comprising the component taken out of the subject is referred to as a liquid extract. The extract and the liquid extract may comprise, for example, the proteoglycan. The extract or the liquid extract may comprise, for example, a component (other component) other than proteoglycan derived from an animal tissue subjected to the extraction. Examples of the other component include lipids, nucleic acids, and proteins.

As used herein, "purification/purified" means identification and separation of a target substance, recovery of the target substance from a component in its natural state, a state in which the target substance has been identified and separated and/or a state in which the target substance has been recovered from the components in its natural state. The "purification" can be carried out by, for example, at least one purification step. The purification may also be referred to as isolation.

Hereinafter, the present disclosure will be described by way of examples, but the present disclosure is not limited to the following examples, and can be implemented with any modifications. The descriptions of the present disclosure and the embodiments can be mutually incorporated unless otherwise specified. **In** this specification, when the expression "to" is used between numerical or physical values, it means that the numerical or physical values before and after "to" are included. **In** the present specification, the expression "A and/or B" means "A alone," "B alone," and "both A and B."

### <Method for Producing Proteoglycan>

In a certain aspect, the present disclosure provides a method for producing proteoglycan. The production method of the present disclosure includes extracting an extract from a tissue of an animal. The proteoglycan comprises a core protein and a sugar chain, and the core protein and the sugar chain are bonded.

In the extracting, an extract comprising proteoglycan is extracted from the animal tissue. Specifically, in the extracting, the animal tissue is brought into contact with an extraction liquid to extract the proteoglycan present in the animal tissue into the extraction liquid. Thus, in the extracting, the proteoglycan can be taken into the extraction liquid, and for example, a liquid extract comprising the proteoglycan can be collected by recovering the extraction liquid. In the extracting, the animal tissue and the extraction liquid can be brought into contact by a known method for bringing a solid and a liquid into contact, particularly by mixing the animal tissue and the extraction liquid.

In general extraction of the proteoglycan from the animal tissue, protease or peptidase such as collagenase and/or a glycolytic enzyme such as cellulase and glucanase, or a metal chelating agent (chelator) such as chondroitinase and EDTA is added to promote the liberation of proteoglycan from the animal tissue. Thus, the core protein and/or the sugar chains of the proteoglycan in the extract or the liquid extract is partially or entirely decomposed. On the other hand, for example, in the extracting, the extract is extracted without degrading the core protein and/or the sugar chains constituting the proteoglycan. That is, the extracting is performed with no exogenous degrading enzyme and/or metal chelating agent comprised or added. Thus, the production method of the present disclosure allows extraction of the proteoglycan with the core protein and the sugar chains bonded, that is, the core protein and/or the sugar chains less degraded. Further, the production method of the present disclosure can extract, for example, proteoglycan having a structure similar to proteoglycan in the animal tissue. Examples of the exogenous degrading enzyme include proteases, peptidases, glycolytic enzymes, and chondroitinases that are not derived from the animal tissue.

The animal may be of any species, and examples thereof include mammalian animals (mammals) such as pigs and cows, avian animals (birds) such as chickens, and fish including *Pleuronectidae* fish such as yellowfin sole, *Salmonidae* fish such as chum salmon and Atlantic salmon, and rays (including, e.g., skates). Preferable examples of the animal include pigs, cows, chickens, flatfishes, salmons, and rays.

The type of GAGs constituting the proteoglycan varies depending on the type of the animal. Thus, in the extracting, the ratio of the disaccharide structures of the GAGs constituting the proteoglycan to be extracted can be controlled by changing the origin of the animal tissue or by mixing tissues derived from different animals. When the animal is a mammal or a bird, the proteoglycan derived from the animal comprises, for example, a relatively large amount of GAGs having a type A or iA disaccharide structure compared to proteoglycans derived from other animals. As a specific example, proteoglycan derived from porcine bronchial cartilage comprises 40% to 60% of the type A disaccharide structure and 10% to 30% of the type iA disaccharide structure, for example. Proteoglycan derived from bovine abomasum comprises 5% to 25% of the type A disaccharide structure and 30% to 60% of the type iA disaccharide structure, for example. Proteoglycan derived from bird (chicken) breast cartilage comprises 50% to 75% of the type A disaccharide structure and 3% to 10% of the type iA disaccharide structure, for example. When extracting the proteoglycan comprising the GAGs having the type A or iA disaccharide structure in a higher ratio, the tissue derived from the animal, which is the mammal and/or the bird, is used or added. This can increase the content ratio of the GAGs having the type A or iA disaccharide structure in the sugar chain of the proteoglycan obtained by the extracting. When the animal is fish, the proteoglycan derived from the animal comprises, for example, a relatively small amount of GAGs having the type A or iA disaccharide structure compared to proteoglycans of other animals. As a specific example, proteoglycan derived from the nasal bone of the chum salmon comprises 10% to 30% of the type A disaccharide structure and 5% to 12.5% of the type iA disaccharide structure, for example. Proteoglycan derived from the fin of yellowfin sole comprises 25% to 45% of the type A disaccharide structure and 3% to 10% of the type iA disaccharide structure, for example. Proteoglycan derived from the nasal cartilage of Atlantic salmon comprises 16% to 26% of the type A disaccharide structure and 2% to 13% of the type iA disaccharide structure, for example. Proteoglycan derived from the fin of ray comprises 5% to 15% of the type A disaccharide structure and 4% to 13% of the type iA disaccharide structure, for example. When extracting the proteoglycan comprising the GAGs having the type A or iA disaccharide structure in a lower ratio, the tissue derived from the animal, which is the fish, is used or added. This can lower the content ratio of the GAGs having the type A or iA disaccharide structure in the sugar chain of the proteoglycan obtained by the extracting.

When the animal is the fish such as the chum salmon, the Atlantic salmon, the yellowfin sole, or the ray, for example, the proteoglycan derived from the animal comprises a relatively large amount of GAGs having a type C or iC disaccharide structure compared to proteoglycans derived from other animals. As a specific example, proteoglycan derived from the nasal cartilage of the chum salmon comprises 45% to 65% or 45% to 55% of the type C disaccharide structure and 0% to 13% or 3% to 13% of the type iC disaccharide structure, for example. Proteoglycan derived from the fin of the yellowfin sole comprises 30% to 50% of the type C disaccharide structure and 0% to 10% of the type iC disaccharide structure, for example. Proteoglycan derived from the nasal cartilage of Atlantic salmon comprises 42% to 52% of the type C disaccharide structure and 1% to 12% of the type iC disaccharide structure, for example. Proteoglycan derived from the fin of the ray (skate) comprises 50% to 70% of the type C disaccharide structure and 0% to 10% of the type iC disaccharide structure, for example. When extracting the proteoglycan comprising the GAGs having the type C or iC disaccharide structure in a higher ratio, the tissue derived from the animal, which is the fish, is used. This can lower the content ratio of the GAGs having the type C or iC disaccharide structure in the sugar chain of the proteoglycan obtained by the extracting. When the animal is a mammal or a bird, the proteoglycan derived from the animal comprises, for example, a relatively small amount of GAGs having a type C or iC disaccharide structure compared to proteoglycans derived from other animals. As a specific example, proteoglycan derived from the porcine bronchial cartilage comprises 10% to 30% of the type C disaccharide structure and 1% to 5% of the type iC disaccharide structure, for example. Proteoglycan derived from the bovine abomasum comprises 10% to 30% (preferably 10% to 25%) of the type C disaccharide structure and 10% or less of the type iC disaccharide structure, for example. Proteoglycan derived from the chicken breast cartilage comprises 15% to 35% of the type C disaccharide structure and 0% to 3% of the type iC disaccharide structure, for example. When extracting the proteoglycan comprising the GAGs having the type C or iC disaccharide structure in a lower ratio, the tissue derived from the animal, which is the mammal or the bird, is used. This can lower the content ratio of the GAGs having the type C or iC disaccharide structure in the sugar chain of the proteoglycan obtained by the extracting.

The content ratio of the disaccharide structures in the proteoglycan can be calculated by analyzing the composition of the disaccharide structures of the proteoglycan, that is, the compositions of the disaccharide structures of the GAGs in the proteoglycan. Specifically, the disaccharide structures of the sugar chains of the proteoglycan can be measured in the manner described in Examples 1(2) and 1(3) shown later by referring to, for example, the measurement methods of the following References 1 and 2.
Reference 1: Takeda Naoko et al., "Facile analysis of contents and compositions of the chondroitin sulfate/dermatan sulfate hybrid chain in shark and ray tissues." Carbohydrate research vol. 424 (2016): 54-8. doi: 10.1016/j.carres.2016.02.006
Reference 2: Tamura, Jun-ichi et al., "Sulfation patterns and the amounts of chondroitin sulfate in the diamond squid, Thysanoteuthis rhombus." Bioscience, biotechnology, and biochemistry vol. 73,6 (2009): 1387-91. doi: 10.1271/bbb.90037

Prior to the measurement of the disaccharide structure, the GAGs are prepared from the proteoglycans. Specifically, the GAGs in the target proteoglycans are purified in the manner described in Example 1(2) shown later. Next, in the manner described in Example 1(3) shown later, unsaturated disaccharides are prepared from the obtained GAGs, and the content ratio of each disaccharide structure is calculated from the HPLC peak area of the obtained unsaturated disaccharide. That is, the peak area of each disaccharide structure in the total peak area measured by HPLC is calculated as the content ratio of each disaccharide structure.

### (Measurement Conditions of HPLC)

Preparation of samples: sugar chains (50 µg) are diluted (to pH 8.0) with H₂O (50 µl), a BSA solution (0.05 mg/5 µl), and a 250 mmol/l Tris-HCl (AcOH) buffer (10 µl), and then digested with chondroitinase ABC (25 mU/25 µl) or chondroitinase AC (25 mU/25 µl) at 37°C or 30°C for eight hours to prepare unsaturated disaccharides.
HPLC system: LC-10AD (manufactured by Shimadzu Corporation)
Column: PA-03 (PA-G) column
Eluent: aqueous NaH₂PO₄ solution
Flow rate: 1.0 ml/min

The molecular weight of the proteoglycan (peak top molecular weight) and the molecular weight of the GAGs (peak top molecular weight) vary depending on the type of the animal. Therefore, in the extracting, the molecular weights of the proteoglycan to be extracted and the GAGs constituting the proteoglycan can be controlled by changing the origin of the animal tissue or mixing animal tissues derived from different animals. As a specific example, the chum salmon-derived proteoglycan has a molecular weight of, for example, 300,000 to 800,000 or 600,000 to 750,000. The chum salmon-derived GAGs have a molecular weight of, for example, 60,000 to 100,000 or 70,000 to 90,000. The Atlantic salmon-derived proteoglycan has a molecular weight of, for example, 1,000,000 to 1,300,000 or 1,100,000 to 1,250,000. The Atlantic salmon-derived GAGs have a molecular weight of, for example, 30,000 to 150,000 or 50,000 to 80,000. The yellowfin sole-derived proteoglycan has a molecular weight of, for example, 600,000 to 1,000,000 or 800,000 to 900,000. The yellowfin sole-derived GAGs have a molecular weight of, for example, 50,000 to 110,000 or 70,000 to 100,000. The ray-derived GAGs have a molecular weight of, for example, 140,000 to 280,000 or 190,000 to 230,000. The chicken-derived proteoglycan has a molecular weight of, for example, 500,000 to 750,000 or 600,000 to 700,000. The chicken-derived GAGs have a molecular weight of, for example, 30,000 to 90,000 or 40,000 to 70,000. The porcine-derived proteoglycan has a molecular weight of, for example, 500,000 to 750,000 or 600,000 to 700,000. The porcine-derived GAGs have a molecular weight of, for example, 10,000 to 50,000 or 20,000 to 40,000. The bovine-derived proteoglycan has a molecular weight of, for example, 300,000 to 600,000 or 400,000 to 500,000. The bovine-derived GAGs have a molecular weight of, for example, 5,000 to 300,000 or 6,000 to 250,000.

In the present specification, the molecular weight of the proteoglycan and the molecular weight of the GAGs may be, for example, a peak top molecular weight measured by gel permeation chromatography (GPC). The molecular weight of the proteoglycan can be measured by GPC in the manner described in Example 1(1) shown later. GPC is performed, for example, under the following conditions, and the molecular weight can be calculated by injecting the following standard samples (molecular weight markers, pullulan) individually into an HPLC system to obtain a molecular weight calibration curve.

### (Conditions for Measurement of Peak Top Molecular Weight (Mp))

HPLC system: LC-10AD (manufactured by Shimadzu Corporation)
Column: TSKgel G5000-PWXL, ø7.8 mm × 300 mm (manufactured by Tosoh Corporation)
Eluent: pH 6.8 phosphate buffer
Flow rate: 0.5 ml/min
Column temperature: 40°C
Detector: refractive index detector (RID-10A manufactured by Shimadzu Corporation)
Injection volume: 50 µl
Molecular weight marker: Shodex STANDARD P-82 (pullulan)

The molecular weight markers have the peak top molecular weight (Mp) and the ratio of a weight average molecular weight (Mw) to a number average molecular weight (Mn) described below.
STD P-800: Mp: 739,000, Mw/Mn: 1.24
STD P-400: Mp: 348,000, Mw/Mn: 1.33
STD P-200: Mp: 216,000, Mw/Mn: 1.22
STD P-100: Mp: 107,000, Mw/Mn: 1.12
STD P-50: Mp: 49,400, Mw/Mn: 1.08
STD P-20: Mp: 22,000, Mw/Mn: 1.08
STD P-10: Mp: 9,800, Mw/Mn: 1.07
STD P-5: Mp: 6,300, Mw/Mn: 1.09

The molecular weight of the GAGs can be measured by, for example, GPC in the manner described in Example 1(2) shown later. GPC is performed, for example, under the following conditions, and the molecular weight can be calculated by injecting the standard samples (molecular weight markers, pullulan) individually into the HPLC system to obtain a molecular weight calibration curve.

### (Conditions for Measurement of Peak Top Molecular Weight of GAG (Mp))

HPLC system: LC-10AD (manufactured by Shimadzu Corporation)
Sample: GAGs (50 µg) diluted in H₂O (50 µg)
Column: Linear coupled size exclusion columns (OHpak SB-G 6B 100 mm × 4.6 mm and SB-805 HQ 250 mm × 4.6 mm)
Eluent: 0.1 mol/l NaNO₃
Flow rate: 1 ml/min

The molecular weight of the proteoglycan and the molecular weight of the GAGs may be number average molecular weights. The number average molecular weight can be measured by GPC in the manner described in Example 1(1) shown later, as in the case of the peak top molecular weight. The chum salmon-derived GAGs have a number average molecular weight of, for example, 20,000 to 130,000 or 40,000 to 110,000. The Atlantic salmon-derived GAGs have a number average molecular weight of, for example, 10,000 to 120,000 or 30,000 to 100,000. The yellowfin sole-derived GAGs have a number average molecular weight of, for example, 30,000 to 140,000 or 50,000 to 120,000. The ray-derived GAGs have a number average molecular weight of, for example, 230,000 to 340,000 or 250,000 to 320,000. The chicken-derived GAGs have a number average molecular weight of, for example, 10,000 to 110,000 or 20,000 to 80,000. The porcine-derived GAGs in the pig-derived proteoglycan have a number average molecular weight of, for example, 10,000 to 60,000 or 20,000 to 40,000.

The molecular weight of the proteoglycan and the molecular weight of the GAGs may be weight average molecular weights. The weight average molecular weight can be measured by GPC in the manner described in Example 1(1) shown later, as in the case of the peak top molecular weight. The chum salmon-derived GAGs have a weight average molecular weight of, for example, 80,000 to 190,000 or 100,000 to 170,000. The Atlantic salmon-derived GAGs have a weight average molecular weight of, for example, 60,000 to 170,000 or 90,000 to 140,000. The yellowfin sole-derived GAGs have a weight average molecular weight of, for example, 90,000 to 200,000 or 110,000 to 170,000. The ray-derived GAGs have a weight average molecular weight of, for example, 370,000 to 480,000 or 390,000 to 460,000. The chicken-derived GAGs have a weight average molecular weight of, for example, 40,000 to 150,000 or 60,000 to 140,000. The porcine-derived GAGs in the pig-derived proteoglycan have a weight average molecular weight of, for example, 10,000 to 110,000 or 30,000 to 90,000.

The animal tissue is, for example, a tissue comprising proteoglycan. Examples of the tissue include: epithelial tissues such as skin; cartilage tissues such as cartilage; digestive organs; circulatory organs; respiratory organs; and placenta. Specific examples of the animal tissue include cartilage, fins, digestive organs, circulatory organs, respiratory organs, and ears. The type of chondroitin sulfate constituting the proteoglycan varies depending on the type of the animal tissue. The animal tissue rich in proteoglycan comprising a relatively large amount of GAGs having the type A and C disaccharide structures than the type iA and iC proteoglycans includes, for example, a cartilage tissue. When extracting the proteoglycan comprising GAGs having the type A or C disaccharide structure in a higher ratio, the cartilage tissue is used as the animal tissue. This can increase the content ratio of the GAGs having the type A or C disaccharide structure in the sugar chain of the proteoglycan obtained by the extracting.

The animal tissue rich in proteoglycan comprising a relatively large amount of GAGs having the type iA and iC disaccharide structures than the type A and C proteoglycans includes, for example, the digestive organ. When extracting the proteoglycan comprising the GAGs having the type iA or iC disaccharide structure in a higher ratio, the digestive organ is used as the animal tissue. This can increase the content ratio of the GAGs having the type iA or iC disaccharide structure in the sugar chain of the proteoglycan obtained by the extracting.

Examples of the extraction liquid used for the extracting include an aqueous guanidine hydrochloride solution, an aqueous acetic acid solution, an aqueous urea solution, and an aqueous magnesium chloride solution. The concentration of the aqueous guanidine hydrochloride solution is, for example, 3 mol/l to 5 mol/l (hereinafter, mol/l also be referred to as "M"). The concentration of the aqueous acetic acid solution is, for example, preferably 3 M to 5 M.

The extraction liquid may comprise other components, for example, to the extent that the proteoglycan with the core protein and the sugar chains bonded can be extracted and/or safety is assured when the extraction liquid is used in vivo. Examples of the other components include protease inhibitors, peptidase inhibitors, glycolytic enzyme inhibitors such as cellulase inhibitors and glucanase inhibitors, and chondroitinase inhibitors. The other components may be used alone or in combination of two or more of them.

The extraction liquid is preferably made to keep the core protein and/or the sugar chains in the proteoglycan from degrading, for example. The extraction liquid does not comprise, for example, exogenous proteases, peptidases, glycolytic enzymes such as cellulases, and chondroitinases. The term "exogenous" means, for example, that the substance is derived from species different from the target animal tissue. The extraction liquid preferably does not comprise, for example, molecules that catalyze hydrolysis of the core protein and/or the sugar chains in the proteoglycan. Examples of the molecules that catalyze the hydrolysis include hydrochloric acid.

In the extracting, the animal tissue and the extraction liquid may be used in any ratio. The amount (volume) of the extraction liquid is, for example, 1 times to 10 times (w/v), preferably 3 times to 7 times (w/v), the amount (mass) of the animal tissue. As a specific example, when the extraction liquid of three times the amount of 1 g of the animal tissue is used, the amount of the extraction liquid is 3 ml, for example.

The extracting can be performed for any amount of time (extraction time) as long as the time is within a range that allows the extraction of the proteoglycan having the core protein and the sugar chains bonded. The extraction time is, for example, one day to two weeks, preferably about seven days.

The extracting can be performed at any temperature (extraction temperature) as long as the temperature is within a range that allows the extraction of an extract comprising proteoglycan with the core protein and the sugar chains bonded. The extraction temperature is, for example, 0°C to 10°C, preferably 0°C to 5°C, more preferably 4°C.

The extracting can be performed at any pH (extraction pH) as long the pH is within a range that allows the extraction of an extract comprising proteoglycan with the core protein and the sugar chains bonded. The extraction pH is, for example, pH 2 to pH 9, preferably pH 6.

During the extracting, for example, stirring may be simultaneously performed to the extent that allows the extraction of an extract comprising proteoglycan with the core protein and the sugar chains bonded.

The molecular weight of the proteoglycan obtained by the extracting, the type of the GAGs comprised in the proteoglycan, and the molecular weight of the GAGs are not limited to particular values, and the proteoglycan may have the molecular weights in desired numerical ranges.

The proteoglycan in the animal tissue usually has a molecular weight of, for example, 400,000 or more. Thus, when the molecular weight of the proteoglycan obtained by the extracting is 400,000 or more, preferably 600,000 or more, it can be evaluated that the core protein and the sugar chains in the proteoglycan are bonded, and the core protein and/or the sugar chains are less degraded.

The proteoglycan obtained by the extracting has a molecular weight of, for example, 400,000 to 1,500,000, 600,000 to 1,500,000, 400,000 to 1,200,000, or 600,000 to 1,200,000. The molecular weights of the proteoglycans derived from the tissues of different animals can be determined by, for example, the same method as described above for the molecular weight of the proteoglycan.

The GAGs (sugar chains) constituting the proteoglycan obtained by the extracting have a molecular weight of, for example, 20,000 to 250,000 or 26,000 to 224,000, preferably 26,000 to 75,000 or 196,000 to 224,000. The molecular weight of the GAGs constituting the proteoglycans derived from tissues of different animals can be determined by, for example, the same method as described above for the molecular weight of GAGs constituting the proteoglycan.

The GAGs (sugar chains) constituting the proteoglycan may include, for example, the type A and/or iA type disaccharide structure. In this case, the proteoglycan obtained by the extracting may comprise the type A and/or iA disaccharide structure in a higher ratio than the other disaccharide structures. As a specific example, the proteoglycan comprises, for example, 20% to 90%, preferably 30% to 70%, of the type A and/or iA disaccharide structure. The proteoglycan comprises the type A disaccharide structure (A) and the type iA disaccharide structure (iA) in a ratio (A:iA) of, for example, 1:1 to 20:1, preferably 1.62:1 to 10.2:1. The ratio (A:iA) varies depending on the origin of the proteoglycan. When the proteoglycan is derived from a pig ear, the ratio (A:iA) is, for example, 0.5:1 to 4:1, 1:1 to 2:1, preferably about 1.62:1. When the proteoglycan is derived from the porcine bronchial cartilage, the ratio (A:iA) is, for example, 1:1 to 5:1, 2:1 to 3:1, preferably about 2.6:1. When the proteoglycan is derived from a pig ear, the ratio (A:iA) is, for example, 3:1 to 4:1, preferably about 3.52:1. When the proteoglycan is derived from the chicken breast cartilage, the ratio (A:iA) is, for example, 7:1 to 13:1, 9:1 to 11:1, preferably about 10.2:1.

The sugar chains of the proteoglycan may include, for example, the type C and/or iC disaccharide structure. In this case, the proteoglycan obtained by the extracting may comprise the type C and/or iC disaccharide structure in a higher ratio than the other disaccharide structures. As a specific example, the proteoglycan comprises 20% to 60% of the type C and/or iC disaccharide structure, for example. The type C disaccharide structure (C) and the type iC disaccharide structure (iC) are in a ratio (C:iC) of, for example, 4:1 to 100:0, preferably 7:1 to 100:0 or 7.46:1 to 100:0. The ratio (C:iC) varies depending on the origin of the proteoglycan. When the proteoglycan is derived from the nasal cartilage of the chum salmon, the ratio (C:iC) is, for example, 5:1 to 10:1, 7:1 to 8:1, preferably about 7.46:1. When the proteoglycan is derived from the whole fin of the ray, the ratio (C:iC) is, for example, 20:1 to 30:1, 23:1 to 24:1, preferably about 23.8:1. When the proteoglycan is derived from the fin root or fin cartilage of the ray or the fin of the yellowfin sole, the ratio (C:iC) is, for example, 90:10 to 100:0 or 95:5 to 100:0.

The GAGs (sugar chains) constituting the proteoglycan obtained by the extracting comprise, for example, 5% to 75% of the type A disaccharide structure, 3% to 30% of the type iA disaccharide structure, 10% to 80% of the type C disaccharide structure, and 0% to 13% of the type iC disaccharide structure. In this case, the molecular weight of the proteoglycan is, for example, 600,000 to 1,500,000 or 600,000 to 1,200,000. The molecular weight of the GAGs is, for example, 26,000 to 224,000, preferably 26,000 to 75,000 or 196,000 to 224,000. In the composition of the present disclosure, the total content of the type A, iA, C, and iC disaccharide structures is 100% or less (the same applies hereinafter).

The GAGs constituting the proteoglycan obtained by the extracting comprise, for example, 40% to 60% of the type A disaccharide structure, 10% to 30% of the type iA disaccharide structure, 10% to 30% of the type C disaccharide structure, and 1% to 5% of the type iC disaccharide structure. In this case, the molecular weight of the proteoglycan is, for example, 500,000 to 750,000 or 600,000 to 700,000. The molecular weight of the GAGs is, for example, 10,000 to 50,000 or 20,000 to 40,000.

The GAGs constituting the proteoglycan obtained by the extracting comprise, for example, 50% to 75% of the type A disaccharide structure, 3% to 10% of the type iA disaccharide structure, 15% to 35% of the type C disaccharide structure, and 0% to 3% of the type iC disaccharide structure. In this case, the molecular weight of the proteoglycan is, for example, 500,000 to 750,000 or 600,000 to 700,000. The molecular weight of the GAGs is, for example, 30,000 to 90,000 or 40,000 to 70,000.

The GAGs constituting the proteoglycan obtained by the extracting comprise, for example, 10% to 30% of the type A disaccharide structure, 5% to 12.5% of the type iA disaccharide structure, 45% to 55% of the type C disaccharide structure, and 3% to 13% of the type iC disaccharide structure, or 10% to 30% of the type A disaccharide structure, 5% to 12.5% of the type iA disaccharide structure, 45% to 65% of the type C disaccharide structure, and 0% to 13% of the type iC disaccharide structure. The molecular weight of the proteoglycan is, for example, 300,000 to 800,000 or 600,000 to 750,000. The molecular weight of the GAGs is, for example, 60,000 to 100,000 or 70,000 to 90,000.

The GAGs constituting the proteoglycan obtained by the extracting comprise, for example, 25% to 45% of the type A disaccharide structure, 3% to 10% of the type iA disaccharide structure, 30% to 50% of the type C disaccharide structure, and 0% to 10% of the type iC disaccharide structure. In this case, the molecular weight of the proteoglycan is, for example, 600,000 to 1,000,000 or 800,000 to 900,000. The molecular weight of the GAGs is, for example, 50,000 to 110,000 or 70,000 to 100,000.

The GAGs constituting the proteoglycan obtained by the extracting comprise, for example, 16% to 26% of the type A disaccharide structure, 2% to 13% of the type iA disaccharide structure, 42% to 52% of the type C disaccharide structure, and 1% to 12% of the type iC disaccharide structure. In this case, the molecular weight of the proteoglycan is, for example, 1,000,000 to 1,300,000 or 1,100,000 to 1,250,000. The molecular weight of the GAGs is, for example, 30,000 to 150,000 or 50,000 to 80,000.

The GAGs constituting the proteoglycan obtained by the extracting comprise, for example, 5% to 15% of the type A disaccharide structure, 4% to 13% of the type iA disaccharide structure, 50% to 70% of the type C disaccharide structure, and 0% to 10% of the type iC disaccharide structure.

The GAGs constituting the proteoglycan obtained by the extracting comprise, for example, 5% to 25% of the type A disaccharide structure, 30% to 60% of the type iA disaccharide structure, 10% to 30% (preferably 10% to 25%) of the type C disaccharide structure, and 10% or less of the type iC disaccharide structure, more preferably 5% to 25% of the type A disaccharide structure, 30% to 60% of the type iA disaccharide structure, 10% to 25% of the type C disaccharide structure, and 10% or less of the type iC disaccharide structure. In this case, the molecular weight of the proteoglycan is, for example, 300,000 to 600,000 or 400,000 to 500,000. The molecular weight of the GAG is, for example, 5,000 to 300,000 or 6,000 to 250,000.

After the extracting, for example, the extract may be filtered. The extract may be filtered by any method, for example, a known solid-liquid separation method. Specifically, the filtration may be performed using a filtration means such as a filter, or solid-liquid separation may be performed by centrifugation. The filtration may be performed once or more.

The method for producing the proteoglycan of the present disclosure may include purifying the extract to obtain a purified substance comprising proteoglycan. The purification can be performed by, for example, bringing the filtered extract into contact with a solvent in which proteoglycan is less soluble to precipitate or deposit the proteoglycan. Thus, the purifying can also be referred to as depositing or precipitating proteoglycan in the extract, for example. The solvent may be an organic solvent including alcohols such as ethanol and methanol. In the purifying, for example, the precipitate or the deposit may further be centrifuged to recover the precipitate or the deposit.

The method for producing the proteoglycan of the present disclosure may include separating a fraction of proteoglycan satisfying a predetermined peak top molecular weight from the purified substance. The separation may be performed by any method as long as the fraction of the proteoglycan with the core protein and the sugar chains bonded can be separated. The separation may be performed by, for example, a known method capable of separating a fraction of a component based on the molecular weight. Specifically, the separation may be performed using an ultrafiltration membrane (molecular weight cut-off membrane) or a dialysis membrane, or by silica gel column chromatography, gel filtration chromatography, or ion exchange column chromatography. The molecular weight cut-off membrane can have a molecular weight cut-off value that is set for the target molecular weight, for example. For the separation, centrifugation may be performed using a container having the molecular weight cut-off membrane. When the centrifugation is performed, for example, a membrane Amicon (registered trademark) Ultra centrifugal filter unit manufactured by Merck Millipore can be used as the container.

In the separating, the purified substance from which the fraction is separated is preferably in a liquid state, for example. Thus, if the purified substance obtained by the purifying is solid, the purified substance is preferably dispersed in a solvent prior to the separating. The solvent may be any solvent as long as it can dissolve proteoglycan with the core protein and the sugar chains bonded. Examples of the solvent include a buffer such as a phosphate buffer, purified water, and an aqueous solvent such as an aqueous sodium acetate solution. The solvent has pH 4 to pH 9, for example.

The method for producing the proteoglycan of the present disclosure may include recovering a solid fraction of the proteoglycan with the core protein and the sugar chains bonded from the fraction comprising the proteoglycan. The recovery can be performed by any method as long as the proteoglycan can be recovered with the core protein and the sugar chains bonded. The recovery can be performed by, for example, a known method capable of recovering a solid fraction of the proteoglycan from the liquid comprising the proteoglycan, and specifically, can be performed by drying such as freeze drying or atomization drying (spray drying).

**In** the production method of the present disclosure, the animal tissue may be pretreated before the extracting. Examples of the pretreatment include pulverization and defatting of the animal. In the production method of the present disclosure, the animal tissue is preferably pulverized to make a contact area between the animal tissue and the extraction liquid relatively large and to extract the proteoglycan more efficiently. The pulverization can be performed by, for example, cutting, crushing, or an ultrasonic treatment. Specifically, the pulverization includes, for example, mechanical crushing using a homogenizer and crushing by an ultrasonic treatment. Thus, the production method of the present disclosure may optionally include the pulverization of the animal tissue prior to the extracting. When a non-pulverized animal tissue is used in the extracting of the production method of the present disclosure, the animal tissue and the extraction liquid are brought into contact, and then the obtained mixture may be pulverized in the extracting.

### <Composition>

In another aspect, the present disclosure provides a composition comprising proteoglycan. The composition of the present disclosure comprises proteoglycan. The proteoglycan has a peak top molecular weight of, for example, 400,000 to 1,200,000. The composition of the present disclosure can promote, for example, proliferation of cells, particularly skin fibroblasts.

The composition of the present disclosure comprises, for example, proteoglycan obtained by the method for producing proteoglycan of the present disclosure. For the composition of the present disclosure, for example, the description of the method for producing proteoglycan of the present disclosure can be referred to.

The GAGs (sugar chains) constituting the proteoglycan comprised in the composition of the present disclosure comprise, for example, 5% to 75% of the type A disaccharide structure, 3% to 30% of the type iA disaccharide structure, 10% to 80% of the type C disaccharide structure, and 0% to 13% of the type iC disaccharide structure. In this case, the molecular weight of the proteoglycan is, for example, 600,000 to 1,500,000 or 600,000 to 1,200,000. The molecular weight of the GAGs is, for example, 26,000 to 224,000, preferably 26,000 to 75,000 or 196,000 to 224,000. In the composition of the present disclosure, the total content of the type A, iA, C, and iC disaccharide structures is 100% or less (the same applies hereinafter).

The GAGs constituting the proteoglycan comprised in the composition of the present disclosure comprise, for example, 40% to 60% of the type A disaccharide structure, 10% to 30% of the type iA disaccharide structure, 10% to 30% of the type C disaccharide structure, and 1% to 5% of the type iC disaccharide structure. In this case, the molecular weight of the proteoglycan is, for example, 500,000 to 750,000 or 600,000 to 700,000. The molecular weight of the GAGs is, for example, 10,000 to 50,000 or 20,000 to 40,000.

The GAGs constituting the proteoglycan comprised in the composition of the present disclosure comprise, for example, 50% to 75% of the type A disaccharide structure, 3% to 10% of the type iA disaccharide structure, 15% to 35% of the type C disaccharide structure, and 0% to 3% of the type iC disaccharide structure. In this case, the molecular weight of the proteoglycan is, for example, 500,000 to 750,000 or 600,000 to 700,000. The molecular weight of the GAGs is, for example, 30,000 to 90,000 or 40,000 to 70,000.

The GAGs constituting the proteoglycan comprised in the composition of the present disclosure comprise, for example, 10% to 30% of the type A disaccharide structure, 5% to 12.5% of the type iA disaccharide structure, 45% to 55% of the type C disaccharide structure, and 3% to 13% of the type iC disaccharide structure, or 10% to 30% of the type A disaccharide structure, 5% to 12.5% of the type iA disaccharide structure, 45% to 65% of the type C disaccharide structure, and 0% to 13% of the type iC disaccharide structure. The molecular weight of the proteoglycan is, for example, 300,000 to 800,000 or 600,000 to 750,000. The molecular weight of the GAGs is, for example, 60,000 to 100,000 or 70,000 to 90,000.

The GAGs constituting the proteoglycan comprised in the composition of the present disclosure comprise, for example, 25% to 45% of the type A disaccharide structure, 3% to 10% of the type iA disaccharide structure, 30% to 50% of the type C disaccharide structure, and 0% to 10% of the type iC disaccharide structure. In this case, the molecular weight of the proteoglycan is, for example, 600,000 to 1,000,000 or 800,000 to 900,000. The molecular weight of the GAGs is, for example, 50,000 to 110,000 or 70,000 to 100,000.

The GAGs constituting the proteoglycan comprised in the composition of the present disclosure comprise, for example, 16% to 26% of the type A disaccharide structure, 2% to 13% of the type iA disaccharide structure, 42% to 52% of the type C disaccharide structure, and 1% to 12% of the type iC disaccharide structure. In this case, the molecular weight of the proteoglycan is, for example, 1,000,000 to 1,300,000 or 1,100,000 to 1,250,000. The molecular weight of the GAGs is, for example, 30,000 to 150,000 or 50,000 to 80,000.

The GAGs constituting the proteoglycan comprised in the composition of the present disclosure comprise, for example, 5% to 15% of the type A disaccharide structure, 4% to 13% of the type iA disaccharide structure, 50% to 70% of the type C disaccharide structure, and 0% to 10% of the type iC disaccharide structure. The molecular weight of the GAGs is, for example, 140,000 to 280,000 or 190,000 to 230,000.

The GAGs constituting the proteoglycan comprised in the composition of the present disclosure comprise, for example, 5% to 25% of the type A disaccharide structure, 30% to 60% of the type iA disaccharide structure, 10% to 30% (preferably 10% to 25%) of the type C disaccharide structure, and 10% or less of the type iC disaccharide structure, more preferably 5% to 25% of the type A disaccharide structure, 30% to 60% of the type iA disaccharide structure, 10% to 25% of the type C disaccharide structure, and 10% or less of the type iC disaccharide structure. In this case, the molecular weight of the proteoglycan is, for example, 300,000 to 600,000 or 400,000 to 500,000. The molecular weight of the GAG is, for example, 5,000 to 300,000 or 6,000 to 250,000.

As used herein, to "promote cell proliferation" means to promote or enhance the proliferation of cells, particularly human-derived cells. The promotion of the cell proliferation can be checked by, for example, evaluating the proliferation of the cells in the manner described in Example 1(4) shown later. The cells may be any cells, and are, for example, cells derived from a skin tissue, preferably skin fibroblasts.

Use of the composition of the present disclosure for, for example, an administration target, can promote the proliferation of skin fibroblasts. The conditions for using the composition of the present disclosure (administration conditions) are not limited to particular ones. For example, the administration form, administration timing, and dosage can be appropriately set for the type of the administration target.

The composition of the present disclosure may be used, for example, in vivo or in vitro.

The composition of the present disclosure can be administrated to any target. When the composition of the present disclosure is used in vivo, the administration target is, for example, humans or non-human animals other than humans. Examples of the non-human animals include mammals such as mice, rats, rabbits, dogs, sheep, horses, cats, goats, monkeys, and guinea pigs, and birds. When the composition of the present disclosure is used in vitro, examples of the administration target include cells, tissues, and organs. Examples of the cells include cells collected from a living body and cultured cells, and examples of the tissues or the organs include tissues (biological tissues) or organs collected from a living body.

In a topical composition for skin (e.g., a composition for transdermal administration or application to the skin) or a composition for oral administration comprising the proteoglycan of the present disclosure as will be described later, the proteoglycan is blended to the extent that the proliferation of the cells, particularly the skin fibroblasts, is well promoted, that is, in an effective amount.

Oral or parenteral administration can be employed to administer the composition of the present disclosure. Examples of the parenteral administration include transdermal administration and application to (contact with) the skin. The application to the skin may include application to oral mucosa, that is, application to or contact with epithelial cells in the oral cavity. The application to the skin may also include, in addition to or instead of the application to the skin surface, intradermal or subcutaneous administration or injection through the skin surface. The intradermal administration or injection through the skin surface can be carried out with a microneedle, for example.

The composition of the present disclosure can take any dosage form, and the dosage form can be appropriately determined for the administration form, for example. The dosage form may be, for example, liquid or solid. For the oral administration, the dosage form can be tablets, pills, capsules, granules, powders, and liquid.

The composition of the present disclosure may comprise, for example, an additive as required, and the additive preferably includes a pharmaceutically acceptable additive or carrier. Any type of additives can be used, and examples thereof include a base material, an excipient, a coloring agent, a lubricant, a binder, a disintegrating agent, a stabilizer, a coating agent, a preservative, and a flavoring agent such as a flavor.

Examples of the excipient include organic excipients including: sugar derivatives such as lactose, lactose hydrate, sucrose, glucose, mannitol, and sorbitol; starch derivatives such as corn starch, potato starch, α-starch, and dextrin; cellulose derivatives such as crystalline cellulose; gum arabic; dextran; and pullulan, and inorganic excipients including silicate derivatives such as light silicic anhydride, synthetic aluminum silicate, calcium silicate, and magnesium metasilicate aluminate; phosphates such as calcium hydrogen phosphate; carbonates such as calcium carbonate; and sulfates such as calcium sulfate. Examples of the coloring agent include yellow ferric oxide. Examples of the lubricant include: stearic acid, metal stearates such as calcium stearate and magnesium stearate; talc; polyethylene glycol; silica; and hydrogenated vegetable oil. Examples of the flavoring agent include flavors such as cocoa powder, menthol, aromatic powder, peppermint oil, borneol, and cinnamon powder, sweeteners, and acidulants. Examples of the binder include hydroxypropyl cellulose, hydroxypropyl methylcellulose, polyvinylpyrrolidone, and macrogol. Examples of the disintegrating agent include cellulose derivatives such as carboxymethyl cellulose and calcium carboxymethyl cellulose; chemically modified starches such as carboxymethyl starch, sodium carboxymethyl starch, crosslinked polyvinylpyrrolidone, and sodium starch glycolate, and chemically modified celluloses. Examples of the stabilizer include: para-hydroxybenzoate esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid. Examples of the coating agent include hypromellose, macrogols such as macrogol 6000, talc, and titanium oxide.

When the composition of the present disclosure is orally administered, specific examples of the composition for oral administration include beverages, foods, pharmaceutical product(s), and quasi-drug(s).

When the composition of the present disclosure is used for transdermal administration or application to the skin (hereinafter, also referred to as "topical skin preparation"), the topical skin preparation may take the form of an ampoule, a capsule, powders, granules, liquid, gel, bubbles, emulsion, a sheet, a mist, or a spray, depending on the form of use. The form of use includes, for example, pharmaceutical product(s), quasi-drug(s), topical skin preparations for local or systemic use, medicinal and/or cosmetic preparations to be applied to the scalp and the hair, bath preparations to be put into bath water for use, and other preparations. Examples of the topical skin preparations for local or systemic use include skin care products such as toners, emulsions, creams, ointments, lotions, oils, and facial masks, facial or skin cleansers such as bar soap, liquid soap, and hand soap, massage agents, cleansing agents, depilatories, epilatories, shaving treatment agents, aftershave lotions, preshave lotions, shaving creams, makeup cosmetics such as foundations, lipsticks, blushers, eye shadows, eyeliners, and mascaras, perfumes, nail care products, nail polishes, nail polish removers, cataplasms, plasters, tapes, sheets, patches, aerosols, toothpastes, and gargles such as mouthwashes. Examples of the medicinal and/or cosmetic preparations to be applied to the scalp and the hair include shampoos, conditioners, hair treatments, hair pretreatments, permanent solutions, hair dyes, hair styling products, hair tonics, hair restoring/nourishing agents, cataplasms, plasters, tapes, sheets, and aerosols. Examples of the other preparations include axillary odor inhibitors or deodorants, antiperspirants, sanitary products, sanitary cottons, and wet wipes.

The topical skin preparation can be produced by selecting and/or combining optional components and/or additives listed below as necessary to the extent that they do not interfere with the effect of promoting the proliferation of the skin fibroblasts.

### (1) Various oils and fats

Examples of the oils and fats include avocado oil, almond oil, fennel oil, perilla oil, olive oil, orange oil, orange roughy oil, sesame oil, cacao butter, camomile oil, carrot oil, cucumber oil, tallowate, kukui nut oil, safflower oil, shea butter, liquid shea butter, soybean oil, camellia oil, corn oil, rapeseed oil, persic oil, castor oil, cottonseed oil, peanut oil, turtle oil, mink oil, egg yolk oil, palm oil, palm kernel oil, Japan wax, coconut oil, beef tallow, lard, squalene, squalane, pristane, and hydrogenated products (hydrogenated oil, etc.) of these oils and fats.

### (2) Waxes

Examples of the waxes include beeswax, carnauba wax, spermaceti, lanolin, liquid lanolin, reduced lanolin, hard lanolin, candelilla wax, montan wax, shellac wax, or rice wax.

### (3) Mineral oils

Examples of the mineral oils include liquid paraffin, petroleum jelly, paraffin, ozokerite, ceresin, and microcrystalline wax.

### (4) Fatty Acids

Examples of the fatty acids include natural fatty acids such as lauric acid, myristic acid, palmitic acid, stearic acid, behenic acid, oleic acid, linoleic acid, linolenic acid, docosahexaenoic acid, eicosapentaenoic acid, 12-hydroxystearic acid, undecylenic acid, tall oil, and lanolin fatty acid, and synthetic fatty acids such as isononanoic acid, caproic acid, 2-ethylbutanoic acid, isopentanoic acid, 2-methylpentanoic acid, 2-ethylhexanoic acid, and isopentanoic acid.

### (5) Alcohols

Examples of the alcohols include natural alcohols such as ethanol, isopropanol, lauryl alcohol, cetanol, stearyl alcohol, oleyl alcohol, lanolin alcohol, cholesterol, phytosterol, and phenoxyethanol, and synthetic alcohols such as 2-hexyldecanol, isostearyl alcohol, and 2-octyldodecanol.

### (6) Polyhydric alcohols

Examples of the polyhydric alcohols include ethylene oxide, ethylene glycol, diethylene glycol, triethylene glycol, ethylene glycol monoethyl ether, ethylene glycol monobutyl ether, diethylene glycol monomethyl ether, diethylene glycol monoethyl ether, polyethylene glycol, propylene oxide, propylene glycol, polypropylene glycol, 1,3-butylene glycol, pentyl glycol, glycerin, pentaerythritol, threitol, arabitol, xylitol, ribitol, galactitol, sorbitol, mannitol, lactitol, and maltitol.

### (7) Esters

Examples of the esters include isopropyl myristate, isopropyl palmitate, butyl stearate, hexyl laurate, myristyl myristate, oleyl oleate, decyl oleate, octyldodecyl myristate, hexyldecyl dimethyloctanoate, cetyl lactate, myristyl lactate, diethyl phthalate, dibutyl phthalate, lanolin acetate, ethylene glycol monostearate, propylene glycol monostearate, and propylene glycol dioleate.

### (8) Metallic Soaps

Examples of the metallic soaps include aluminum stearate, magnesium stearate, zinc stearate, calcium stearate, zinc palmitate, magnesium myristate, zinc laurate, and zinc undecylenate.

### (9) Gums, Sugars or Water-Soluble Polymers

Examples of the gums, sugars, and water-soluble polymers include Gum arabic, benzoin gum, dammar gum, guaiac gum, Irish moss, karaya gum, tragacanth gum, carob gum, quince seed, agar, casein, lactose, fructose, saccharose or esters thereof, trehalose or derivatives thereof, dextrin, gelatin, pectin, starch, carrageenan, carboxymethylchitin or chitosan, hydroxyalkyl (C2 to C4) chitin or chitosan to which alkylene (C2 to C4) oxide such as ethyleneoxide is added, low-molecular chitin or chitosan, chitosan salts, sulfated chitin or chitosan, phosphorylated chitin or chitosan, alginic acid or salts thereof, hyaluronic acid or salts thereof, chondroitin sulfate or salts thereof, heparin, ethylcellulose, methylcellulose, carboxymethylcellulose, carboxyethyl cellulose, sodium carboxyethyl cellulose, hydroxyethylcellulose, hydroxypropyl cellulose, nitrocellulose, crystalline cellulose, polyvinyl alcohol, polyvinyl methyl ether, polyvinylpyrrolidone, polyvinyl methacrylate, polyacrylates, polyalkylene oxide such as polyethylene oxide and polypropylene oxide or crosslinked polymers thereof, carboxyvinyl polymers, and polyethyleneimine.

### (10) Surfactants

Examples of the surfactants include anionic surfactants (alkyl carboxylates, alkyl sulfonates, alkyl sulfate ester salts, and alkyl phosphate ester salts), cationic surfactants (alkylamine salts and alkyl quaternary ammonium salts), amphoteric surfactants such as carboxylate type amphoteric surfactants (amino type and betaine type), sulfate type amphoteric surfactants, sulfonic acid type amphoteric surfactants, and phosphate type amphoteric surfactants, nonionic surfactants (ether type nonionic surfactants, ether ester type nonionic surfactants, ester type nonionic surfactants, block polymer type nonionic surfactants, and nitrogen-comprising nonionic surfactants), and other surfactants (natural surfactants, derivatives of protein hydrolysates, polymer surfactants, surfactants comprising titanium and silicon, and fluorocarbon surfactants).

### (11) Vitamins

Examples of the vitamins include: vitamins A such as retinol, retinal (vitamin A1), dehydroretinal (vitamin A2), carotenes, and lycopene (provitamin A); vitamins B such as thiamin hydrochloride, thiamin sulphate (vitamin B1), riboflavine (vitamin B2), pyridoxine (vitamin B6), cyanocobalamin (vitamin B12), folic acids, nicotinic acids, pantothenic acids, biotins, choline, and inositols; vitamins C such as vitamin C acid or derivatives thereof; vitamins D such as ergocalciferol (vitamin D2), cholecalciferol (vitamin D3), and dihydrotachysterol; vitamins E such as vitamin E or derivatives thereof, and ubiquinones; vitamins K such as phytonadione (vitamin K1), menaquinone (vitamin K2), menadione (vitamin K3), and menadiol (vitamin K4); and essential fatty acids (vitamin F), carnitine, ferulic acid, γ-oryzanol, orotic acid, vitamins P (rutin, eriocitrin, hesperidin), and vitamin U.

### (12) Various Amino Acids

Examples of the amino acids include valine, leucine, isoleucine, threonine, methionine, phenylalanine, tryptophan, lysine, glycine, alanine, asparagine, glutamine, serine, cysteine, cystine, tyrosine, proline, hydroxyproline, aspartic acid, glutamic acid, hydroxylysine, arginine, ornithine, histidine, and their sulfates, phosphates, nitrates, citrates, and amino acid derivatives such as pyrrolidone carboxylic acid.

### (13) Additives

The topical skin preparation may further comprise various additives derived from animals or plants. The additive can be any additive selected from various materials and processed by a common method suitable for the type and form of a product to which the additive is to be added. The additive can be processed by, for example, any of crushing, milling, washing, hydrolysis, fermentation, purification, compression, extraction, fractionation, filtration, drying, powdering, granulation, dissolution, sterilization, pH adjustment, deodorization, and decolorization optionally selected and/or combined.

A solvent used for the extraction can be selected in consideration of the purpose of use and the kind of product to be provided, or a processing treatment to be performed later. The solvent for the extraction is preferably one or a mixture of two or more selected from organic solvents such as water, lower alcohols or hydrous lower alcohols such as methanol, ethanol, propyl alcohol, isopropyl alcohol, butanol, and isobutanol, polyhydric alcohols or hydrous polyhydric alcohols such as propylene glycol, 1,3-butylene glycol, and glycerin, acetone, and ethyl acetate. If it is not preferable for the extraction solvent to comprise the organic solvent depending on the use, for example, water alone may be used, or ethanol which is easily removed after the extraction may be used alone or as a mixture with water in any ratio. Alternatively, an additive extracted by squeezing may be used.

When the plant or animal material-derived additive is used for topical preparations or cosmetics for systemic or local use, the topical skin preparations are expected to have cosmetic effects of, for example, protecting and moisturizing the skin and the hair, improving their feel and texture, softening the skin and the hair, stimulation relief, stress relief by aroma, cell activation (anti-aging of cells), inhibiting inflammation, improving skin and hair quality, reducing skin roughness and improving skin texture, helping hair growth and restoration, reducing hair loss, making the skin and the hair shiny, cleansing, relieving fatigue, promotion of blood circulation, and improved bath effect, and other effects such as the effects of perfuming, deodorizing, thickening, antisepsis, and buffering.

In expectation of various cosmetic and pharmaceutical effects of the materials known in the art other than those mentioned above, the materials can be combined to reinforce the effect of the present disclosure, providing the topical skin preparation with multi-functional effects.

### <Method for Promoting Cell Proliferation>

In another aspect, the present disclosure discloses a method capable of promoting cell proliferation. The method for promoting the cell proliferation of the present disclosure uses the composition of the present disclosure. The method for promoting the cell proliferation of the present disclosure is expected to have the effect of promoting the cell proliferation, for example.

The method for promoting the cell proliferation of the present disclosure includes use of the composition of the present disclosure on a target. The use may be, for example, bringing the composition into contact with the skin or administration of the composition to the skin.

In the method for promoting the cell proliferation of the present disclosure, the use may be, for example, in vitro or in vivo. For the target (administration target) of the method for promoting the cell proliferation of the present disclosure and the administration conditions, for example, the description of the administration target and the administration conditions related to the composition of the present disclosure can be referred to.

The cells may be any cells, and are, for example, cells derived from a skin tissue, preferably skin fibroblasts.

### <Use>

The present disclosure is directed to a composition or use of the composition to promote the cell proliferation. The present disclosure is directed to the use of a composition for the production of a composition used to promote the cell proliferation.

### Examples

Examples of the present disclosure will be described below. Note that the present disclosure is not limited to the following examples. Commercial reagents were used according to their protocols unless otherwise indicated. Note that "mol/l" may be denoted as "M."

### [Example 1]

It was confirmed that the proteoglycan of the present disclosure with the core protein and the sugar chains bonded could be produced by the production method of the present disclosure.

### (1) Purification of Proteoglycan

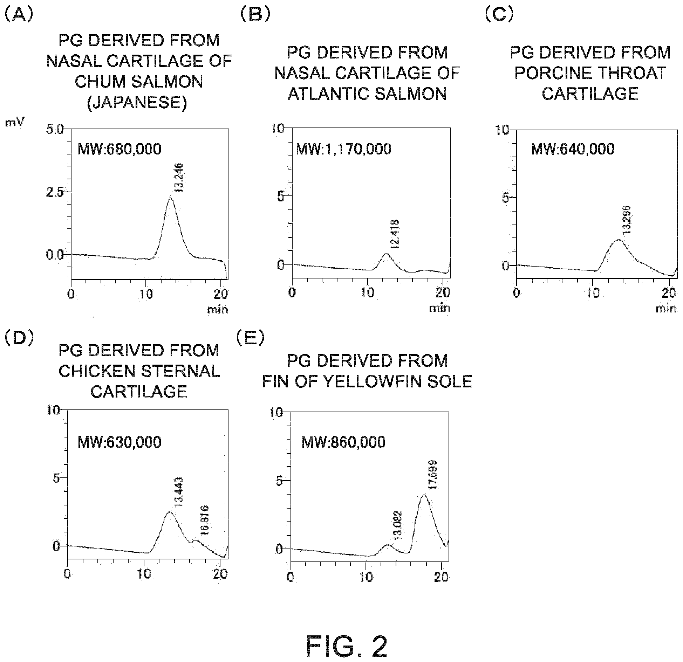

As starting materials, nasal cartilage of chum salmon, nasal cartilage of Atlantic salmon, porcine throat cartilage, sternal cartilage (breast cartilage) of chicken, and the fin of yellowfin sole, 100 g to 150 g each, were prepared. To each of the starting materials, five times (W/V) the amount of a 4 M aqueous guanidine hydrochloride solution was added. After the addition, immersion extraction was performed at 4°C for a week. The obtained extract was filtered with a 60 mesh sieve and filter paper (3 µm). Three times (v/v) the amount of ethanol was added to the obtained filtrate. After the addition, the obtained mixture was allowed to stand at 4°C overnight. After the standing, the mixture was filtered with filter paper (3 µm) to recover the precipitate. A phosphate buffer (pH 6.8) was added to the recovered precipitate to dissolve the precipitate. After the dissolution, fractionation purification was performed using a molecular weight cut-off membrane (MW: 50,000, manufactured by Asahi Kasei Corporation). After the fractionation purification, a fraction of proteoglycan was recovered. The recovered proteoglycan fraction was freeze-dried. After the freeze-drying, a proteoglycan solid was obtained. The proteoglycan solid was analyzed by HPLC under the following measurement conditions. (A) to (E) in FIG. 2 and Table 1 show the results.

### (Measurement Conditions of HPLC)

HPLC system: LC-10AD
Column: TSKgel G5000-PWXL ø7.8 mm × 300 mm (manufactured by Tosoh Corporation) Eluent: pH 6.8 phosphate buffer
Flow rate: 0.5 ml/min
Column temperature: 40°C
Detector: refractive index detector (RID-10A manufactured by Shimadzu Corporation)
Injection volume: 50 µl
Molecular weight marker: Shodex STANDARD P-82 (pullulan)

In FIG. 2, (A) to (E) are graphs of the results of the HPLC analyses of the proteoglycan solids. In FIG. 2, the vertical axis represents peak intensity (mV), and the horizontal axis represents retention time (min). As shown in (A) of FIG. 2, proteoglycan eluted from the nasal cartilage of the cham salmon at around 13.246, and the peak top molecular weight was 680,000. As shown in (B) of FIG. 2, proteoglycan eluted from the nasal cartilage of the Atlantic salmon at around 12.418, and the peak top molecular weight was 1,170,000. As shown in (C) of FIG. 2, proteoglycan eluted from the porcine throat cartilage at around 13.296, and the peak top molecular weight was 640,000. As shown in (D) of FIG. 2, proteoglycan eluted from the chicken sternal cartilage at around 13.443 and 16.816, and the peak top molecular weight obtained by combining the two peaks was 630,000. As shown in (E) of FIG. 2, proteoglycan eluted from the fin of the yellowfin sole at around 13.082 and 17.699, and the peak top molecular weight obtained by combining the two peaks was 860,000.

**[Table 1]**

| | Chum salmon | Atlantic salmon | Pig | Chicken | Yellowfin sole |
|---|---|---|---|---|---|
| PG purity (%) | 42.7 | 22.5 | 101.0 | 108.8 | 77.7 |
| Solid content yield (%) | 2.62 | 2.39 | 0.26 | 1.04 | 1.14 |

Table 1 shows proteoglycan purities (%) and solid content yields (%) of the proteoglycan solids. As shown in Table 1, the nasal cartilage of the chum salmon had a proteoglycan purity of 42.7% and a solid content yield of 2.62%. As shown in Table 1, the nasal cartilage of the Atlantic salmon had a proteoglycan purity of 22.5% and a solid content yield of 2.39%. As shown in Table 1, the porcine throat cartilage had a proteoglycan purity of 101.0% and a solid content yield of 0.26%. As shown in Table 1, the chicken sternal cartilage had a proteoglycan purity of 108.8% and a solid content yield of 1.04%. As shown in Table 1, the fin of the yellowfin sole had a proteoglycan purity of 77.7% and a solid content yield of 1.14%.

### (2) Analysis of GAGs in Proteoglycans

GAGs in the proteoglycans obtained in Example 1(1) were analyzed. Specifically, the proteoglycans obtained in Example 1(1) were purified to obtain the GAGs. The proteoglycans obtained in Example 1(1), 10 g each, were treated with 80 ml of boiling water for 10 minutes. To each proteoglycan after the treatment, 100 ml of a 0.5 mol/l borate buffer (pH 7.0) was added. After the addition, 0.67 g of protease N Amano G (manufactured by Amano Enzyme Inc.) was added, and the mixture was incubated at 55°C for seven days. The incubated mixture was quenched with 5% trichloroacetic acid. The quenched mixture was filtered to remove insoluble matters and precipitated with 80% ethanol comprising 1.25% NaOAc (sodium acetate). After the precipitation, the mixture was centrifuged at 0°C, and the obtained precipitate was vacuum-dried to obtain crude sugar chains. Then, 100 mg of the crude sugar chains was diluted with 0.05 mol/l acetic acid (pH 4.0) comprising 0.15 mol/l LiCl. The diluted solution was added to a DEAE-cellulose column (ø2.2 cm × 15 cm, manufactured by Wako Pure Chemical Cooperation). After the addition, the column was washed stepwise with a 160 ml buffer comprising 0.15 mol/l, 0.5 mol/l, 1.0 mol/l, or 2.0 mol/l LiCl. After the washing, a carbazole-positive fraction was recovered and dialyzed, and the dialyzed sample was desalted with a gel permeation column (LH-20, H₂O, ø1.1 cm × 80 cm) to obtain GAGs. Then, 50 µg of the GAGs was diluted with 50 µg of H₂O. The diluted sample was analyzed by HPLC using linear coupled size exclusion columns (OHpak SB-G 6B 100 mm × 4.6 mm and SB-805 HQ 250 mm × 4.6 mm).

### (Measurement Conditions of HPLC)

HPLC system: LC-10AD (manufactured by Shimadzu Corporation)
Columns: OHpak SB-G 6B 100 mm × 4.6 mm and SB-805 HQ 250 mm × 4.6 mm
Eluent: 0.1 mol/l NaNO₃
Flow rate: 1 mL/min

The analysis results showed that the chum salmon-derived GAGs had a peak top molecular weight (Mp) of 49,000, a number average molecular weight (Mn) of 78,000, a weight average molecular weight (Mw) of 137,000, and a polydispersity (d) of 1.75. The Atlantic salmon-derived GAGs had a peak top molecular weight of 44,000, a number average molecular weight of 63,000, a weight average molecular weight of 110,000, and a polydispersity of 1.75. The yellowfin sole-derived GAGs had a peak top molecular weight of 75,000, a number average molecular weight of 87,000, a weight average molecular weight of 141,000, and a polydispersity of 1.61. The chicken-derived GAGs had a peak top molecular weight of 49,000, a number average molecular weight of 53,000, a weight average molecular weight of 92,000, and a polydispersity of 1.74. The porcine-derived GAGs had a peak top molecular weight of 26,000, a number average molecular weight of 32,000, a weight average molecular weight of 61,000, and a polydispersity of 1.94. The ray fin-derived GAGs had a peak top molecular weight of 224,000, a number average molecular weight of 281,000, a weight average molecular weight of 423,000, and a polydispersity of 1.51. Table 2 below shows the peak top molecular weights and other values of the GAGs measured.

**[Table 2]**

| Measured part | Molecular weight | | | |
|---|---|---|---|---|
| | Peak top (Mp) | Number average molecular weight (Mn) | Weight average molecular weight (Mw) | Polydispersity (d) |
| Chum salmon | 49,000 | 78,000 | 137,000 | 1.75 |
| Atlantic salmon | 44,000 | 63,000 | 110,000 | 1.75 |
| Yellowfin sole | 75,000 | 87,000 | 141,000 | 1.61 |
| Chicken | 49,000 | 53,000 | 92,000 | 1.74 |
| Pig | 26,000 | 32,000 | 61,000 | 1.94 |
| Ray fin | 224,000 | 281,000 | 423,000 | 1.51 |

### (3) Disaccharide Structure of Each Proteoglycan

For the GAGs obtained in Example 1(2), disaccharide structures of the proteoglycans were analyzed. Specifically, 50 µg of each GAG obtained in Example 1(2) was diluted with 50 µl of H₂O, a BSA solution (0.05 mg/5 µl), and 10 µl of a 250 mmol/l Tris-HCl (AcOH) solution (pH 8.0). The diluted solution was digested with chondroitinase ABC (25 mU/25 µl, manufactured by Sigma Aldrich) or chondroitinase AC (25 mU/25 µl, manufactured by Sigma Aldrich) at 37°C or 30°C for eight hours. After the digestion, unsaturated disaccharides resulting from the digestion were analyzed by HPLC using a PA-03 (PA-G) column. In the analysis, the content ratio (composition ratio) of each disaccharide structure was calculated based on the HPLC peak area of the unsaturated disaccharide as a standard. Table 3 below shows the results.

**[Table 3]**

| Name | Part | CSDS (%) | | | | | |
|---|---|---|---|---|---|---|---|
| | | O | iO | A | iA | C | iC |
| Chum salmon | Nasal cartilage | 10 | 4.0 | 21 | 7.6 | 47 | 6.3 |
| Pig | Bronchial cartilage | 6.1 | 0 | 52 | 20 | 19 | 2.1 |
| Chicken | Breast cartilage | 5.2 | 0 | 63 | 6.2 | 24 | 0.3 |
| Yellowfin sole | Fin | 14 | 2.1 | 34 | 6.8 | 40 | 0 |
| Ray | Fin root | 8.8 | 2.8 | 11 | 8.6 | 60 | 0 |

As shown in Table 3, the contents of the disaccharide structures were as follows.
- GAGs from the nasal cartilage of the chum salmon
   Chondroitin sulfate O: 10%, chondroitin sulfate iO: 4.0%, chondroitin sulfate A: 21%, chondroitin sulfate iA: 7.6%, chondroitin sulfate C: 47%, chondroitin sulfate iC: 6.3%
- GAGs from the porcine bronchial cartilage
   Chondroitin sulfate O: 6.1%, chondroitin sulfate iO: 0%, chondroitin sulfate A: 52%, chondroitin sulfate iA: 20%, chondroitin sulfate C: 19%, chondroitin sulfate iC: 2.1%
- GAGs from the chicken breast cartilage Chondroitin sulfate O: 5.2%, chondroitin sulfate iO: 0%, chondroitin sulfate A: 63%, chondroitin sulfate iA: 6.2%, chondroitin sulfate C: 24%, chondroitin sulfate iC: 0.3%
- GAGs from the fin of the yellowfin sole
   Chondroitin sulfate O: 14%, chondroitin sulfate iO: 2.1%, chondroitin sulfate A: 34%, chondroitin sulfate iA: 6.8%, chondroitin sulfate C: 40%, chondroitin sulfate iC: 0%
- GAGs from the fin root of the ray
   Chondroitin sulfate O: 8.8%, chondroitin sulfate iO: 2.8%, chondroitin sulfate A: 11%, chondroitin sulfate iA: 8.6%, chondroitin sulfate C: 60%, chondroitin sulfate iC: 0%

The proteoglycans derived from the mammal or the bird were found to comprise relatively more GAGs including the type A or iA disaccharide structures than the fish-derived proteoglycans. Further, the fish-derived proteoglycans were found to comprise relatively more GAGs including the type C or iC disaccharide structures than the proteoglycans derived from the mammal or the bird. The results indicate that the type of GAGs constituting the proteoglycan varies depending on the type of animal from which the proteoglycan is derived.

### (4) Cell Proliferation Effect by Proteoglycan

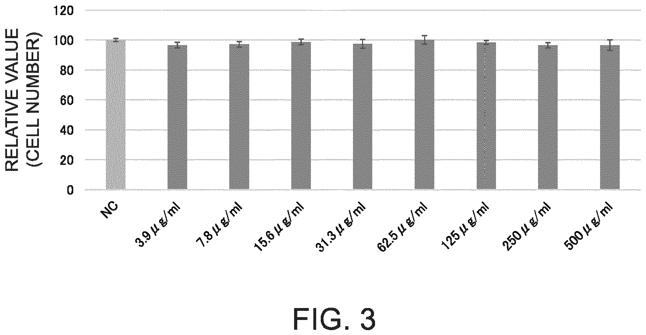

The proteoglycans obtained in Example 1(1) were examined for cell proliferation activity. Specifically, the proteoglycans obtained in Example 1(1) were examined for the cell proliferation activity by comparison with the cell proliferation activity of chondroitin sulfate A. Human dermal fibroblasts (normal human dermal fibroblasts: NHDF, purchased from Kurabo Industries, Ltd.) were cultured in DMEM containing 10% FBS and an antibiotic under the conditions of 37°C and 5% CO₂. After the culture, each proteoglycan obtained in Example 1(1) or chondroitin sulfate A (4SGAG) was added to 1% FBS DMEM, and the cells were cultured. The proteoglycan or 4SGAG was used at a concentration of 3.9 µg/ml, 7.8 µg/ml, 15.6 µg/ml, 31.3 µg/ml, 62.5 µg/ml, 125 µg/ml, 250 µg/ml, or 500 µg/ml. The proteoglycans did not completely dissolve at 500 µg/ml irrespective of their origins, and thus the 500 µg/ml solutions in which the proteoglycans did not completely dissolve were used. The culture was performed for 72 hours. After the culture, the cells were counted using a CCK-8 assay kit (Dojindo Laboratories). As a negative control, the same procedure was carried out except that the proteoglycans obtained in Example 1(1) were not added. As a positive control, the same procedure was carried out except that the proteoglycans obtained in Example 1(1) were replaced with C-028 derived from the salmon nasal cartilage. FIG. 3 and Table 4 show the results.

FIG. 3 is a graph of the results of cell proliferation tests of chondroitin sulfate A. In FIG. 3, the vertical axis represents the relative value of the cell number, and the horizontal axis represents the concentration of chondroitin sulfate A.

The numerical values on the vertical axis of FIG. 3 represent the values of each group, regarding the value of the negative control (NC) group as 100.

The numerical values on the vertical axis of FIG. 3 are as follows.

NC group: 100, 3.9 µg/ml group: 96.5, 7.8 µg/ml group: 97.0, 15.6 µg/ml group: 98.5, 31.3 µg/ml group: 97.3, 62.5 µg/ml group: 100.0, 125 µg/ml group: 98.3, 250 µg/ml group: 96.5, 500 µg/ml group: 96.4

As shown in FIG. 3, compared to the group to which the proteoglycan was not added (negative control), the groups to which chondroitin sulfate A added showed no cell proliferation effect.

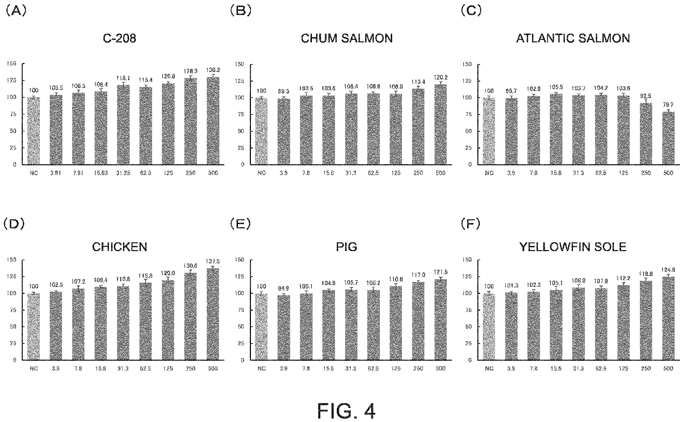

In FIG. 4, (A) to (F) are graphs showing the results of the cell proliferation test of the proteoglycans obtained in Example 1(1). In FIG. 4, the vertical axis represents the relative value of the cell number, and the horizontal axis represents the concentration of proteoglycan.

The numerical values on the vertical axis of FIG. 4 represent the values of each group with respect to the value of the negative control (NC) group as 100.

The numerical values on the vertical axis of (A) in FIG. 4 are as follows.

NC group: 100, 3.91 µg/ml C-028 group: 103.5, 7.81 µg/ml C-028 group: 106.5, 15.63 µg/ml C-028 group: 108.4, 31.25 µg/ml C-028 group: 118.1, 62.5 µg/ml C-028 group: 115.4, 125 µg/ml C-028 group: 120.8, 250 µg/ml C-028 group: 128.3, 500 µg/ml C-028 group: 130.3

The numerical values on the vertical axis of (B) in FIG. 4 are as follows.

NC group: 100, 3.9 µg/ml chum salmon group: 99.3, 7.8 µg/ml chum salmon group: 103.5, 15.6 µg/ml chum salmon group: 103.6, 31.3 µg/ml chum salmon group: 106.4, 62.5 µg/ml chum salmon group: 106.6, 125 µg/ml chum salmon group: 106.0, 250 µg/ml chum salmon group: 113.4, 500 µg/ml chum salmon group: 120.2

The numerical values on the vertical axis of (C) in FIG. 4 are as follows.

NC group: 100, 3.9 µg/ml Atlantic salmon group: 99.7, 7.8 µg/ml Atlantic salmon group: 102.9, 15.6 µg/ml Atlantic salmon group: 105.5, 31.3 µg/ml Atlantic salmon group: 103.7, 62.5 µg/ml Atlantic salmon group: 104.2, 125 µg/ml Atlantic salmon group: 103.6, 250 µg/ml Atlantic salmon group: 92.5, 500 µg/ml Atlantic salmon group: 79.2

The numerical values on the vertical axis of (D) in FIG. 4 are as follows.

NC group: 100, 3.9 µg/ml chicken group: 102.5, 7.8 µg/ml chicken group: 107.2, 15.6 µg/ml chicken group: 109.4, 31.3 µg/ml chicken group: 110.8, 62.5 µg/ml chicken group: 115.8, 125 µg/ml chicken group: 120.0, 250 µg/ml chicken group: 130.6, 500 µg/ml chicken group: 137.5

The numerical values on the vertical axis of (E) in FIG. 4 are as follows.

NC group: 100, 3.9 µg/ml pig group: 96.9, 7.8 µg/ml pig group: 100.1, 15.6 µg/ml pig group: 104.8, 31.3 µg/ml pig group: 105.7, 62.5 µg/ml pig group: 105.2, 125 µg/ml pig group: 110.6, 250 µg/ml pig group: 117.0, 500 µg/ml pig group: 121.5

The numerical values on the vertical axis of (E) in FIG. 4 are as follows.

NC group: 100, 3.9 µg/ml yellowfin sole Group: 101.3, 7.8 µg/ml yellowfin sole Group: 102.3, 15.6 µg/ml yellowfin sole Group: 105.1, 31.3 µg/ml yellowfin sole Group: 108.8, 62.5 µg/ml yellowfin sole Group: 107.8, 125 µg/ml yellowfin sole Group: 112.2, 250 µg/ml yellowfin sole Group: 118.8, 500 µg/ml yellowfin sole Group: 124.8

As shown in (A) of FIG. 4, compared to the group to which the proteoglycan was not added (negative control), the groups to which C-028 (positive control) added showed the cell proliferation activity. As shown in (B) to (F) of FIG. 4, the proteoglycans obtained in Example 1(1), namely, the proteoglycan derived from the nasal cartilage of the chum salmon, the proteoglycan derived from the chicken sternal cartilage, the proteoglycan derived from the porcine throat cartilage, and the proteoglycan derived from the fin of the yellowfin sole showed the cell proliferation effect in a proteoglycan concentration-dependent manner.

The results shown in FIGS. 3 and 4 indicate that chondroitin sulfate A alone that constitutes the proteoglycan did not show the activity of proliferating the skin fibroblasts. In contrast, the proteoglycans produced by the method of the present disclosure with the core protein and the sugar chains bonded showed the activity of proliferating the skin fibroblasts. This indicates that the proteoglycans produced by the method of the present disclosure with the core protein and the sugar chains bonded have the activity of proliferating the skin fibroblasts.

### [Example 2]

The proteoglycans obtained in Example 1 were analyzed for the composition of the GAGs.

### (1) Analysis of GAGs in Proteoglycans

The proteoglycans obtained in Example 1(1) were analyzed for the GAGs. Specifically, from the proteoglycans purified in the same manner as in Example 1(1), the GAGs were purified and analyzed by HPLC in the same manner as in Example 1(2). As the proteoglycans, the proteoglycan derived from the nasal cartilage of the chum salmon (sCSPG), the proteoglycan derived from the chicken breast cartilage (cCSPG), and the proteoglycan derived from the porcine bronchial cartilage of (pCSPG) were used.

As a result, the GAGs (sGAGs) derived from the nasal cartilage of the chum salmon showed the peak top molecular weight (Mp) of 84,000, the GAGs (cGAGs) derived from the chicken breast cartilage showed the peak top molecular weight (Mp) of 77,000, and the GAGs (pGAGs) derived from the porcine bronchial cartilage showed the peak top molecular weight (Mp) of 51,000.

### (3) Disaccharide Structure in Each Proteoglycan

For the GAGs obtained in Example 2(1), the disaccharide structures in the proteoglycans were analyzed. Specifically, the content ratio (composition ratio) of each disaccharide structure was calculated in the same manner as described in Example 1(3). Table 4 below shows the results.

**[Table 4]**

| | CSDS (%) | | | | | |
|---|---|---|---|---|---|---|
| | O | iO | A | iA | C | iC |
| sGAG | 11 | 0 | 27 | 0 | 61 | 0 |
| cGAG | 5.2 | 0 | 63 | 6.5 | 24 | 0.3 |
| pGAG | 6.1 | 0 | 52 | 20 | 19 | 2.1 |

As shown in Table 4, the contents of the disaccharide structures were as follows.
- GAGs derived from the nasal cartilage of the chum salmon (sGAGs), extracted with guanidine Chondroitin sulfate O: 11%, chondroitin sulfate iO: 0%, chondroitin sulfate A: 27%, chondroitin sulfate iA: 0%, chondroitin sulfate C: 61%, chondroitin sulfate iC: 0%
- GAGs derived from the chicken breast cartilage (cGAGs), extracted with guanidine Chondroitin sulfate O: 5.2%, chondroitin sulfate iO: 0%, chondroitin sulfate A: 63%, chondroitin sulfate iA: 6.5%, chondroitin sulfate C: 24%, chondroitin sulfate iC: 0.3%
- GAGs derived from the porcine bronchial cartilage (pGAGs), extracted with guanidine Chondroitin sulfate O: 6.1%, chondroitin sulfate iO: 0%, chondroitin sulfate A: 52%, chondroitin sulfate iA: 20%, chondroitin sulfate C: 19%, chondroitin sulfate iC: 2.1%

### [Example 3]

It was confirmed that the proteoglycan of the present disclosure with the core protein and the sugar chains bonded could be produced by the production method of the present disclosure.

### (1-1) Purification of Bovine-Derived Proteoglycan

As a starting material, about 824 g of abomasum of a cow (Holstein) was prepared. The abomasum was washed and cut into cubes of about 2 cm while removing as much fat as possible. To the cubes, four times the amount of ethanol (3288 ml) was added, and the mixture was allowed to stand for 10 minutes. This process was repeated once. Then, four times the amount of ethanol (3288 ml) was added, and the mixture was allowed to stand overnight. After the standing, ethanol was removed, and the resultant was air-dried. The air-dried product was then dried under reduced pressure to obtain 173 g of a defatted dry product.

### (1-2) Guanidine Extraction

Next, the defatted dry product was purified by guanidine extraction to obtain proteoglycan. Specifically, 87 g of the defatted dry product of Example 3(1-1) was immersed in 350 ml of a 4 M aqueous guanidinium hydrochloride solution for extraction at 4°C for three days. After the immersion extraction, 347 ml of a 4 M aqueous guanidinium hydrochloride solution was added to perform the immersion extraction at 4°C for three days. After the immersion extraction, suction filtration and dialysis were performed. After the dialysis, the dialyzed solution was freeze-dried to obtain 9.93 g of a proteoglycan solid (bovine stomach-derived proteoglycan extracted with guanidine (dermatan sulfate proteoglycan: DSPG)).

### (1-3) Acetic Acid Extraction

In parallel with the guanidine extraction, the defatted dry product was purified by acetic acid extraction to obtain proteoglycan. Specifically, 86 g of the defatted dry product obtained in Example 3(1-1) was immersed in 344 ml of a 4 wt% aqueous acetic acid solution for extraction at 4°C for three days. After the immersion extraction, 344 ml of a 4 wt% aqueous acetic acid solution was added to conduct the immersion extraction at 4°C for another three days. After the immersion extraction, suction filtration and dialysis were performed. After the dialysis, the dialyzed solution was freeze-dried to obtain 0.53 g of a proteoglycan solid (bovine stomach-derived proteoglycan extracted with acetic acid).

### (2-1) Purification of Porcine-Derived Proteoglycan

As a starting material, about 1,282 g of stomach of a pig (six months old) was prepared. The stomach was washed and cut into cubes of about 2 cm while removing as much fat as possible. To the cubes, four times the amount of ethanol (5182 ml) was added, and the mixture was allowed to stand for 10 minutes. This process was repeated once. Then, four times the amount of ethanol (5182 ml) was added, and the mixture was allowed to stand overnight. After the standing, ethanol was removed, and the resultant was air-dried. The air-dried product was then dried under reduced pressure to obtain 261 g of a defatted dry product.

### (2-2) Guanidine Extraction

Next, the defatted dry product was purified by guanidine extraction to obtain proteoglycan. Specifically, 131 g of the defatted dry product obtained in Example 3(2-1) was immersed in 1047 ml of a 4 M aqueous guanidinium hydrochloride solution for extraction at 4°C for six days. After the immersion extraction, suction filtration and dialysis were performed. After the dialysis, the dialyzed solution was freeze-dried to obtain 11.92 g of a proteoglycan solid (porcine stomach-derived proteoglycan extracted with guanidine).

### (2-3) Acetic Acid Extraction

In parallel with the extraction of Example 3(2-2), the defatted dry product was purified by acetic acid extraction to obtain proteoglycan. Specifically, 130 g of the defatted dry product obtained in Example 3(2-1) was immersed in 1040 ml of a 4 wt% aqueous acetic acid solution for extraction at 4°C for six days. After the immersion extraction, suction filtration and dialysis were performed. After the dialysis, the dialyzed solution was freeze-dried to obtain 0.44 g of a proteoglycan solid (porcine stomach-derived proteoglycan extracted with acetic acid).

### (3) Analysis of Disaccharide Structure of Each Proteoglycan

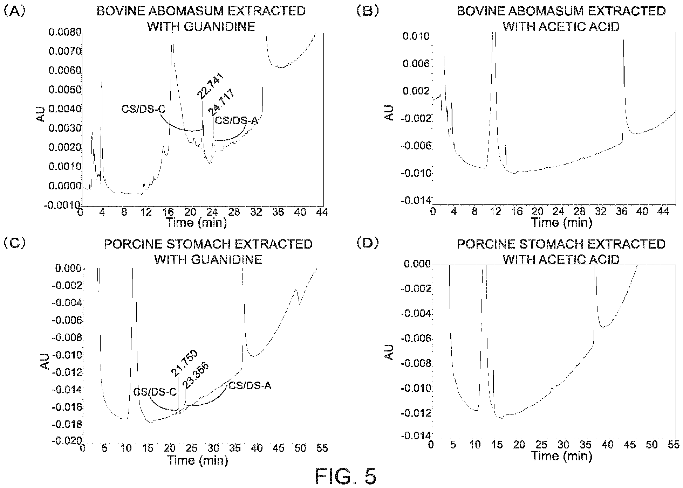

The bovine stomach-derived proteoglycan extracted with guanidine, the bovine stomach-derived proteoglycan extracted with acetic acid, the porcine stomach-derived proteoglycan extracted with guanidine, and the porcine stomach-derived proteoglycan extracted with acetic acid were analyzed for the disaccharide structures. Specifically, the bovine stomach-derived proteoglycan extracted with guanidine of Example 3(1-2), the bovine stomach-derived proteoglycan extracted with acetic acid of Example 3(1-3), the porcine stomach-derived proteoglycan extracted with guanidine of Example 3(2-2), and the porcine stomach-derived proteoglycan extracted with acetic acid of Example 3(2-3) were digested and analyzed by HPLC in the same manner as in Example 1(3). FIG. 5 shows the results.

In FIG. 5, (A) to 5(D) are graphs of the results of HPLC analyses of the disaccharide structures of the proteoglycans. In FIG. 5, the vertical axis represents peak intensity (AU), and the horizontal axis represents retention time (min). In FIG. 5, (A) shows the results of the bovine stomach-derived proteoglycan extracted with guanidine, (B) shows the results of the bovine stomach-derived proteoglycan extracted with acetic acid, (C) shows the results of the porcine stomach-derived proteoglycan extracted with guanidine, and (D) shows the results of the porcine stomach-derived proteoglycan extracted with acetic acid. As shown in FIG. 5, the bovine stomach-derived proteoglycan extracted with guanidine and the porcine stomach-derived proteoglycan extracted with guanidine were found to comprise chondroitin sulfate (CS) and dermatan sulfate (DS).

### (4-1) Salt Exchange of Guanidine-Extracted Proteoglycan with Tris Buffer

When the proteoglycan is purified by guanidine extraction, uronic acid (e.g., glucuronic acid and iduronic acid) of the GAGs constituting the proteoglycan is substituted with guanidine salt. If the guanidine-extracted proteoglycan is used as it is, guanidine which is harmful is liberated. Thus, salt exchange of the guanidine-extracted proteoglycan was performed with a Tris buffer. Specifically, 60 ml of a Tris buffer (20 mmol/l Tris-HCl, 137 mmol/l NaCl) was added to 0.2 g of the bovine stomach-derived proteoglycan of Example 3 (1-2). After the addition, the mixture was sonicated and allowed to stand overnight. After the standing, the mixture was centrifuged to obtain a precipitate and a supernatant. The precipitate and the supernatant were dialyzed and freeze-dried.

### (4-2) Salt Exchange of Guanidine-Extracted Proteoglycan with Aqueous Sodium Bicarbonate Solution

In parallel with the salt exchange with the Tris buffer in Example 3(4-1), salt exchange of the guanidine-extracted proteoglycan with an aqueous sodium bicarbonate solution was performed. Specifically, 30 ml of NaHCO₃ was added to 0.1 g of the bovine stomach-derived proteoglycan obtained in Example 3(1-2). After the addition, the mixture was sonicated and allowed to stand overnight. After the standing, the mixture was centrifuged to obtain a precipitate and a supernatant. The precipitate and the supernatant were dialyzed and freeze-dried.

### (4-3) Analysis of Disaccharide Structure of Each Proteoglycan

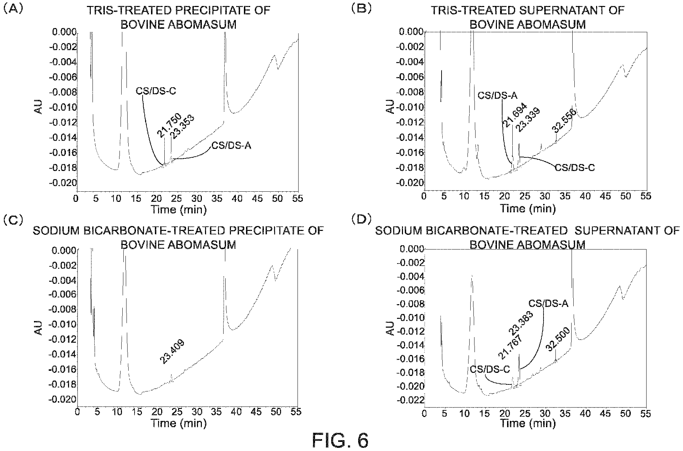

The precipitate and supernatant obtained after the salt exchange were analyzed for disaccharide structure. Specifically, the precipitates and the supernatants obtained after the salt exchange and the freeze-drying in Examples 3(4-1) and 3(4-2) were digested and analyzed by HPLC in the same manner as described in Example 1(3). FIGS. 6 and Table 5 show the results.

In FIG. 6, (A) to (D) are graphs of the results of the HPLC analyses of the disaccharide structures of the proteoglycans. In FIG. 6, the vertical axis represents peak intensity (AU), and the horizontal axis represents retention time (min). In FIG. 6, (A) shows the results of the Tris buffer-treated precipitate, (B) shows the results of the Tris buffer-treated supernatant, (C) shows the results of the precipitate treated with the aqueous sodium bicarbonate solution, and (D) shows the results of the supernatant treated with the aqueous sodium bicarbonate solution. As shown in FIG. 6, the proteoglycans were found to comprise chondroitin sulfate (CS) and dermatan sulfate (DS) after the salt exchange of guanidine.

**[Table 5]**

| | Tris buffer | Aqueous sodium bicarbonate solution |
|---|---|---|
| Supernatant | 182 mg | 190 mg |
| Precipitate | 50 mg | 24 mg |

Table 5 shows the total amount of chondroitin sulfate and dermatan sulfate (CS-DS amount) comprised in the defatted dry product after the salt exchange when converted to 100 g of a defatted dry product of bovine abomasum. As shown in Table 5, the CS-DS amount in the Tris buffer-treated supernatant was 182 mg, the CS-DS amount in the supernatant treated with the aqueous sodium bicarbonate solution was 190 mg, the CS-DS amount in the Tris buffer-treated precipitate was 50 mg, and the CS-DS amount in the precipitate treated with the aqueous sodium bicarbonate solution was 24 mg. Although not shown in Table 4, the proteoglycan comprised in the Tris buffer-treated supernatant had a peak top molecular weight of 460 kDa.

### (5) Detection of Decorin

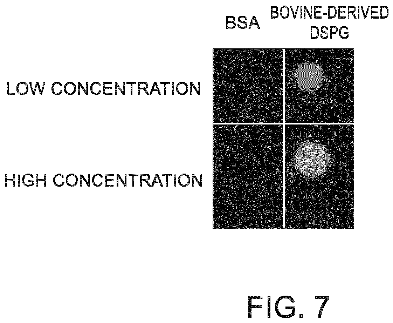

Next, whether decorin is detected in the salt exchanged proteoglycan was examined. Specifically, for the freeze-dried product of the salt-exchanged supernatant obtained in Example 3(4-1) or 3(4-2), reduction and alkylation of proteoglycan was performed using FOCUS Protein Reduction-Alkylation (786 to 231, manufactured by G-Biosciences). To 500 µl of 1 mg/ml proteoglycan that was salt-exchanged with a neutral buffer (pH 7.5), 2.5 µl of a reductant buffer was added, and the mixture was stirred for 10 seconds. After the stirring, 10 ml of FOCUS protein reductant was added, and the mixture was allowed to stand at 55°C for an hour. After the standing, 2.5 ml of an alkylation buffer was added, and the mixture was stirred for 10 seconds. After the stirring, 0.4 mol/l iodoacetamide was added, and the mixture was stirred in a dark place at room temperature (about 25°C, the same applies hereinafter) for an hour to alkylate the sample. Next, a PVDF membrane (Immobilon-FL membrane, manufactured by Merck) was treated with methanol for a minute for hydrophilization. After the hydrophilization, the PVDF membrane was replaced with TBS for five minutes. After the replacement, the PVDF membrane was set in a filtration blotting device (manufactured by Bio-Rad laboratories, Inc.). Then, the alkylated sample was added to the wells, followed by suction. After the suction, the PVDF membrane was removed from the filtration blotting device and allowed to stand and dry at room temperature. After the standing and drying, the PVDF membrane was washed with TBS comprising 0.05% Tween-20 (Tris buffer, TBS-T). After the washing, blocking was performed on the PVDF membrane with an EveryBlot blocking buffer (manufactured by Bio-Rad laboratories, Inc.) for five minutes. After the blocking, a primary antibody (anti-decorin antibody, Cat No: 14667-1-AP, manufactured by Proteintech) diluted to 1/1000 was added for a primary reaction at 4°C for 18 hours. After the primary reaction, the reaction product was washed with TBS-T for five minutes five times. After the washing, StarBright Blue (manufactured by Bio-Rad laboratories, Inc.) diluted to 1/2500 and 0.05% SDS were added for a secondary reaction at room temperature for an hour. After the secondary reaction, the reaction product was washed with TBS-T for five minutes six times. After the washing, the PVDF membrane was washed with ultrapure water and replaced with ultrapure water. After the replacement, fluorescence detection was performed on the PVDF membrane using a multiplex fluorescence detector (ChemiDoc Touch MP, manufactured by Bio-Rad laboratories, Inc.). FIG. 7 shows the results.

FIG. 7 is a photograph showing the results of decorin detection. In FIG. 7, the left column shows the results of BSA, and the right column shows the results of the bovine-derived proteoglycan (DSPG). As shown in FIG. 7, a signal was detected from the bovine-derived DSPG. The results indicate that decorin is retained as the core protein in the bovine-derived proteoglycan extracted with guanidine and salt-exchanged with a neutral buffer (pH 7.5).

Although the present disclosure has been described with reference to the embodiments and the examples, the present disclosure is not limited to the embodiments and the examples. Various modifications that can be understood by those skilled in the art can be made to the configuration and details of the present disclosure within the scope of the present disclosure.

This application claims priority to Japanese Patent Application No. 2022-211853 filed on December 28, 2022, the entire disclosure of which is incorporated herein by reference.

### <Supplementary Note>

Some or all of the above-described embodiments and examples can be modified as in the following supplementary notes, but are not limited to them.

### <Method for Producing Proteoglycan>

### (Supplementary Note 1)

A method for producing proteoglycan, comprising: extracting an extract comprising proteoglycan from a tissue of an animal, the proteoglycan comprising a core protein and a sugar chain, the core protein and the sugar chain being bonded.

### (Supplementary Note 2)

The method of Supplementary Note 1, further comprising purifying the extract to obtain a purified substance comprising the proteoglycan; and separating a fraction of the proteoglycan satisfying a predetermined peak top molecular weight from the purified substance.

### (Supplementary Note 3)

The method of Supplementary Note 2, further comprising: recovering a solid fraction of the proteoglycan from the fraction comprising the proteoglycan.

### (Supplementary Note 4)

The method of any one of Supplementary Notes 1 to 3, wherein the proteoglycan has a peak top molecular weight of 400,000 to 1,200,000.

### (Supplementary Note 5)

The method of any one of Supplementary Notes 1 to 4, wherein the sugar chain of the proteoglycan has a peak top molecular weight of 20,000 to 250,000.

### (Supplementary Note 6)

The method of any one of Supplementary Notes 1 to 5, wherein the sugar chain of the proteoglycan includes a type A and/or iA disaccharide structure, and the proteoglycan comprises the type A and/or iA disaccharide structure in a higher ratio than other disaccharide structures.

### (Supplementary Note 7)

The method of any one of Supplementary Notes 1 to 6, wherein the sugar chain of the proteoglycan includes a type A and/or iA disaccharide structure, and the proteoglycan comprises 20% to 90% of the type A and/or iA disaccharide structure.

### (Supplementary Note 8)

The method of any one of Supplementary Notes 1 to 7, wherein the sugar chain of the proteoglycan includes a type A and/or iA disaccharide structure, and the proteoglycan comprises the type A disaccharide structure (A) and the type iA disaccharide structure (iA) in a ratio (A:iA) of 1:1 to 20:1.

### (Supplementary Note 9)

The method of any one of Supplementary Notes 1 to 8, wherein the sugar chain of the proteoglycan includes a type C and/or iC disaccharide structure, and the proteoglycan comprises the type C and/or iC disaccharide structure in a higher ratio than other disaccharide structures.

### (Supplementary Note 10)

The method of any one of Supplementary Notes 1 to 9, wherein the sugar chain of the proteoglycan includes a type C and/or iC disaccharide structure, and the proteoglycan comprises 20% to 50% of the type C and/or iC disaccharide structure.

### (Supplementary Note 11)

The method of any one of Supplementary Notes 1 to 10, wherein the sugar chain of the proteoglycan includes a type C and/or iC disaccharide structure, and the proteoglycan comprises the type C disaccharide structure (C) and the type iC disaccharide structure (iC) in a ratio (C:iC) of 4:1 to 100:0.

### (Supplementary Note 12)

The method of any one of Supplementary Notes 1 to 11, wherein the animal includes at least one of a mammal or fish.

### (Supplementary Note 13)

The method of any one of Supplementary Notes 1 to 12, wherein the animal is selected from the group consisting of pigs, cows, birds, flatfishes, salmons, and rays.

### (Supplementary Note 14)

The method of any one of Supplementary Notes 1 to 13, wherein the tissue of the animal is selected from the group consisting of cartilage, fins, digestive organs, circulatory organs, respiratory organs, and ears.

### <Composition Comprising Proteoglycan>

### (Supplementary Note 15)

A composition comprising proteoglycan, wherein the proteoglycan has a peak top molecular weight of 400,000 to 1,200,000.

### (Supplementary Note 16)

The composition of Supplementary Note 15, wherein a sugar chain of the proteoglycan has a peak top molecular weight of 20,000 to 250,000.

### (Supplementary Note 17)

The composition of Supplementary Note 15 or 16, wherein a sugar chain of the proteoglycan includes a type A and/or iA disaccharide structure, and the proteoglycan comprises the type A and/or iA disaccharide structure in a higher ratio than other disaccharide structures.

### (Supplementary Note 18)

The composition of any one of Supplementary Notes 15 to 17, wherein a sugar chain of the proteoglycan includes a type A and/or iA disaccharide structure, and a content ratio of the sugar chain having the type A and/or iA disaccharide structure is 20% to 90%.

### (Supplementary Note 19)

The composition of any one of Supplementary Notes 15 to 18, wherein a sugar chain of the proteoglycan includes a type A and/or iA disaccharide structure, and the type A disaccharide structure (A) and the type iA disaccharide structure (iA) are in a ratio (A:iA) of 1:1 to 20:1.

### (Supplementary Note 20)

The composition of any one of Supplementary Notes 15 to 19, wherein a sugar chain of the proteoglycan includes a type C and/or iC disaccharide structure, and the proteoglycan comprises the type C and/or iC disaccharide structure in a higher ratio than other disaccharide structures.

### (Supplementary Note 21)

The composition of any one of Supplementary Notes 15 to 20, wherein a sugar chain of the proteoglycan includes a type C and/or iC disaccharide structure, and the proteoglycan comprises 20% to 50% of the type C and/or iC disaccharide structure.

### (Supplementary Note 22)

The composition of any one of Supplementary Notes 15 to 21, wherein a sugar chain of the proteoglycan includes a type C and/or iC disaccharide structure, and the type C disaccharide structure (C) and the type iC disaccharide structure (iC) are in a ratio (C:iC) of 7:1 to 100:0.

### (Supplementary Note 23)

The composition of any one of Supplementary Notes 15 to 22, wherein the composition is derived from an animal including at least one of a mammal or fish.

### (Supplementary Note 24)

The composition of any one of Supplementary Notes 15 to 23, wherein the composition is derived from an animal selected from the group consisting of pigs, cows, birds, flatfishes, salmons, and rays.

### (Supplementary Note 25)

The composition of any one of Supplementary Notes 15 to 24, wherein the composition is derived from a tissue selected from the group consisting of cartilage, fins, digestive organs, circulatory organs, respiratory organs, and ears.

### (Supplementary Note 26)

The composition of any one of Supplementary Notes 15 to 25 used to promote cell proliferation.

### <Method for Promoting Cell Proliferation>

### (Supplementary Note 27)

A method for promoting cell proliferation, comprising using the composition comprising proteoglycan of any one of Supplementary Notes 15 to 26.

### (Supplementary Note 28)

The method of Supplementary Note 27, comprising using a composition comprising the proteoglycan for a target.

### (Supplementary Note 29)

The method of Supplementary Note 26 or 27, wherein the composition is used in vitro or in vivo.

### <Use>

### (Supplementary Note 30)

Use of the composition comprising proteoglycan of any of Supplementary Notes 15 to 26 to promote cell proliferation.

### INDUSTRIAL APPLICABILITY

As can be seen in the foregoing, the present disclosure can produce proteoglycan in a simpler manner than known methods for producing proteoglycan, and can produce proteoglycan with a core protein and sugar chains bonded. Thus, the present disclosure is extremely useful in, for example, cosmetic and pharmaceutical fields.

## Claims

1. A method for producing proteoglycan, comprising:
extracting an extract comprising proteoglycan from a tissue of an animal,
the proteoglycan comprising a core protein and a sugar chain,
the core protein and the sugar chain being bonded.

2. The method of claim 1, further comprising
purifying the extract to obtain a purified substance comprising the proteoglycan; and
separating a fraction of the proteoglycan satisfying a predetermined peak top molecular weight from the purified substance.

3. The method of claim 2, further comprising
recovering a solid fraction of the proteoglycan from the fraction comprising the proteoglycan.

4. The method of any one of claims 1 to 3, wherein
the proteoglycan has a peak top molecular weight of 400,000 to 1,200,000.

5. The method of any one of claims 1 to 4, wherein
the sugar chain of the proteoglycan has a peak top molecular weight of 20,000 to 250,000.

6. The method of any one of claims 1 to 5, wherein
the sugar chain of the proteoglycan includes a type A and/or iA disaccharide structure, and
the proteoglycan comprises the type A and/or iA disaccharide structure in a higher ratio than other disaccharide structures.

7. The method of claim 6, wherein
the proteoglycan comprises 20% to 90% of the type A and/or iA disaccharide structure.

8. The method of claim 6 or 7, wherein
the proteoglycan comprises the type A disaccharide structure (A) and the type iA disaccharide structure (iA) in a ratio (A:iA) of 1:1 to 20:1.

9. The method of any one of claims 1 to 8, wherein
the sugar chain of the proteoglycan includes a type C and/or iC disaccharide structure, and
the proteoglycan comprises the type C and/or iC disaccharide structure in a higher ratio than other disaccharide structures.

10. The method of claim 9, wherein
the proteoglycan comprises 20% to 50% of the type C and/or iC disaccharide structure.

11. The method of claim 9 or 10, wherein
he type C disaccharide structure (C) and the type iC disaccharide structure (iC) are in a ratio (C:iC) of 4:1 to 100:0.

12. The method of any one of claims 1 to 11, wherein
the animal includes at least one of a mammal or fish.

13. The method of any one of claims 1 to 12, wherein
the animal is selected from the group consisting of pigs, cows, birds, flatfishes, salmons, and rays.

14. The method of any one of claims 1 to 13, wherein
the tissue of the animal is selected from the group consisting of cartilage, fins, digestive organs, circulatory organs, respiratory organs, and ears.

15. A composition comprising proteoglycan, wherein
the proteoglycan has a peak top molecular weight of 400,000 to 1,200,000.

16. The composition of claim 15, wherein
a sugar chain of the proteoglycan has a peak top molecular weight of 20,000 to 250,000.

17. The composition of claim 15 or 16, wherein
a sugar chain of the proteoglycan includes a type A and/or iA disaccharide structure, and
the proteoglycan comprises the type A and/or iA disaccharide structure in a higher ratio than other disaccharide structures.

18. The composition of claim 17, wherein
a content ratio of the sugar chain having the type A and/or iA disaccharide structure is 20% to 90%.

19. The composition of claim 17 or 18, wherein
the type A disaccharide structure (A) and the type iA disaccharide structure (iA) are in a ratio (A:iA) of 1:1 to 20:1.

20. The composition of any one of claims 15 to 19, wherein
a sugar chain of the proteoglycan includes a type C and/or iC disaccharide structure, and
the proteoglycan comprises the type C and/or iC disaccharide structure in a higher ratio than other disaccharide structures.

21. The composition of claim 20, wherein
the proteoglycan comprises 20% to 50% of the type C and/or iC disaccharide structure.

22. The composition of claim 20 or 21, wherein
the type C disaccharide structure (C) and the type iC disaccharide structure (iC) are in a ratio (C:iC) of 7:1 to 100:0.

23. The composition of any one of claims 15 to 22, wherein
the composition is derived from an animal including at least one of a mammal or fish.

24. The composition of any one of claims 15 to 23, wherein
the composition is derived from an animal selected from the group consisting of pigs, cows, birds, flatfishes, salmons, and rays.

25. The composition of any one of claims 15 to 24, wherein
the composition is derived from a tissue selected from the group consisting of cartilage, fins, digestive organs, circulatory organs, respiratory organs, and ears.

26. The composition of any one of claims 15 to 25, used to promote cell proliferation.
